# EUROPÄISCHE PATENTSCHRIFT

(11) **EP 2 029 546 B1**
(45) Veröffentlichungstag und Bekanntmachung des Hinweises auf die Patenterteilung: **28.07.2010**
(21) Anmeldenummer: 07725589.1
(22) Anmeldetag: 25.05.2007
(51) Int. Cl.: C07D 231/12, C07D 401/04, C07D 417/12, C07D 417/14, C07D 419/14, A61P 7/02

(54) **ARYL-SUBSTITUIERTE HETEROZYKLEN UND IHRE VERWENDUNG**
ARYL-SUBSTITUTED HETEROCYCLES, AND USE THEREOF
HÉTÉROCYCLES SUBSTITUÉS PAR UN ARYLE ET LEUR UTILISATION

(30) Priorität: 31.05.2006 DE 102006025314
(43) Veröffentlichungstag der Anmeldung: 04.03.2009
(73) Patentinhaber: Bayer Schering Pharma Aktiengesellschaft, 13353 Berlin (DE)
(72) Erfinder: HÄRTER, Michael, 51375 Leverkusen (DE); WUNBERG, Tobias, 2371 Hinterbrühl (AT); ALLERHEILIGEN, Swen, 45259 Essen (DE); BAUSER, Marcus, 42115 Wuppertal (DE); RESTER, Ulrich, 42115 Wuppertal (DE); HEITMEIER, Stefan, 42489 Wülfrath (DE)
(86) Internationale Anmeldenummer: PCT/EP2007/004693
(87) Internationale Veröffentlichungsnummer: WO 2007/137791

(56) Entgegenhaltungen:
- WO-A-2005/032468

## Beschreibung

Die Erfindung betrifft neue Aryl-substituierte Heterozyklen, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervernetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungskaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77 (online-Publikation August 2004)].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469; A. Casimiro-Garcia et al., "Progress in the discovery of Factor Xa inhibitors" Expert Opin. Ther. Patents 2006, 15, 119-145]. Weiterhin sind nicht-peptidische, niedermolekulare Faktor Xa-Inhibitoren beispielsweise auch in WO 06/002099 und WO 03/026652 beschrieben.

Eine Aufgabe der vorliegenden Erfindung ist die Bereitstellung neuer alternativer Verbindungen mit vergleichbarer oder verbesserter Wirkung, zur Bekämpfung von Erkrankungen, insbesondere von thromboembolischen Erkrankungen, bei Menschen und Tieren.

Gegenstand der Erfindung sind Verbindungen der Formel 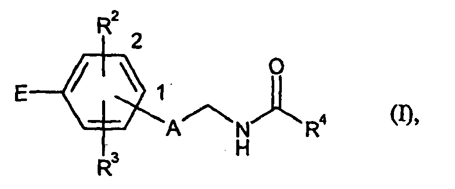 in welcher
- E: für eine Gruppe der Formel 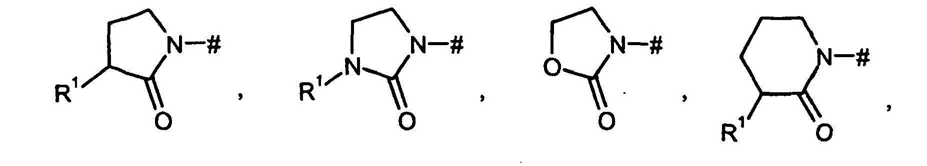 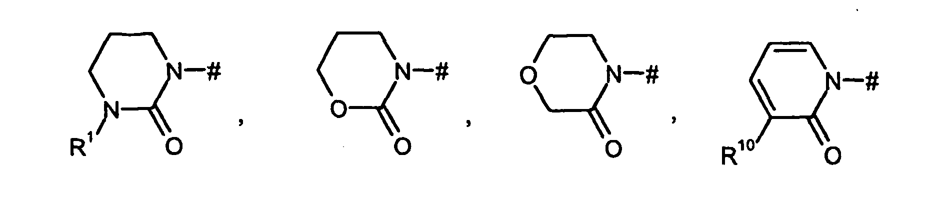 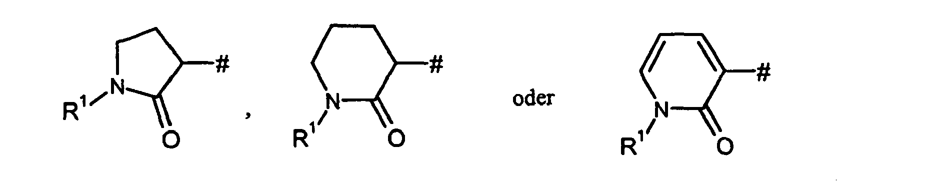 steht,
wobei
# die Anknüpfstelle an den Phenylring ist,
R¹ für Wasserstoff, Hydroxy, Amino, C₁-C₄-Akyl, C₁-C₄-Alkoxy, C₁-C₄-Alkylamino, C₃-C₆-Cycloalkyl, C₃-C₆-Cycloalkyloxy, C₃-C₆-Cycloalkylamino, C₁-C₄-Alkylcarbonylamino oder C₁-C₄-Alkoxycarbonylamino steht,
worin
Alkyl, Alkoxy, Alkylamino substituiert sein können mit einem Substituenten, wobei der Substituent ausgewählt wird aus der Gruppe bestehend aus Hydroxy, Amino, C₁-C₄-Alkoxy, C₁-C₄-Alkylamino, C₃-C₆-Cycloalkylamino und einem 4-bis 7-gliedrigen gesättigten, über ein N-Atom gebundenen Heterocyclyl, das ein Ringglied aus der Reihe N-R⁵ oder O enthalten kann,
worin
R⁵ für Wasserstoff oder C₁-C₄-Alkyl steht, und
R¹⁰ für Wasserstoff, C₁-C₄-Alkyl, C₁-C₄-Alkoxy, C₃-C₆-Cycloalkyl, C₃-C₆-Cycloalkyloxy oder -NR¹¹R¹² steht, wobei
R¹¹ für C₁-C₄-Alkyl oder C₃-C₆-Cycloalkyl steht, und
R¹² für C₁-C₄-Alkyl steht,
- A: für ein 5-gliedriges Heteroaryl oder teilweise ungesättigtes 5-gliedriges Heterocyclyl steht, wobei Heteroaryl und Heterocyclyl in 1 oder 2 Position an den Phenyl-Ring gebunden sind und Heteroaryl und Heterocyclyl selber eine 1,3-Verknüpfung mit dem Phenyl-Ring und der Carbonylaminomethyl-Gruppe aufweisen,
und
wobei Heteroaryl und Heterocyclyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl oder ein 5- oder 6-gliedriges Heteroaryl steht,
wobei Phenyl und Heteroaryl substituiert sein können mit 1 bis 3 Substituenten, wobei die Substituenten unabhängig voneinander ausgewählt werden aus der Gruppe bestehend aus Halogen, Hydroxy, Hydroxymethyl, Hydroxyethyl, Amino, Aminomethyl, Aminoethyl, C₁-C₄-Alkyl, C₁-C₄-Alkoxy, C₁-C₄-Alkoxymethyl, C₁-C₄-Alkylamino, C₁-C₄-Alkylaminomethyl, Hydroxycarbonyl, Hydroxycarbonylmethyl, Aminocarbonyl, Aminocarbonylmethyl, C₁-C₄-Alkoxycarbonyl, C₁-C₄-Alkoxycarbonylmethyl, C₁-C₄-Alkylaminocarbonyl, C₁-C₄-Alkylaminocarbonylmethyl, Aminosulfonyl, C₁-C₄-Alkylaminosulfonyl und C₁-C₄-Alkylsulfonyl,
- R²: für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkoxymethyl, C₁-C₄-Alkylamino, C₃-C₆-Cycloalkyl, Aminocarbonyl, C₁-C₄-Alkoxycarbonyl oder C₁-C₄-Alkylaminocarbonyl steht,
- R³: für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkoxymethyl, C₁-C₄-Alkylamino, C₃-C₆-Cycloalkyl, Aminocarbonyl, C₁-C₄-Alkoxycarbonyl oder C₁-C₄-Alkylaminocarbonyl steht,
- R⁴: für eine Gruppe der Formel 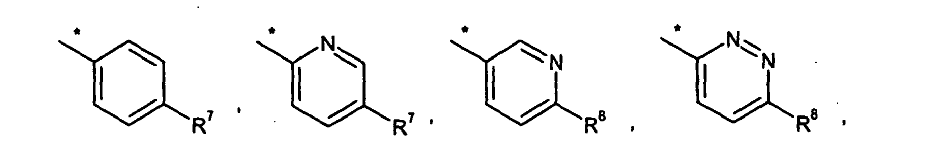 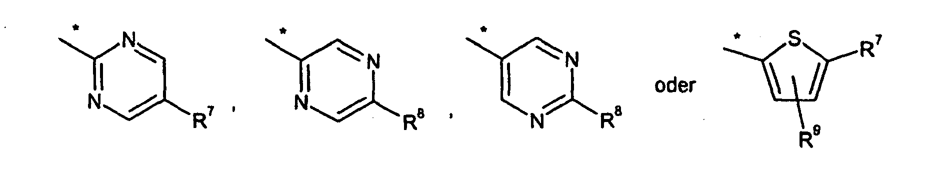 steht,
wobei
* die Anknüpfstelle an die Carbonylgruppe ist,
R⁷ für Wasserstoff, Fluor, Chlor, Cyano, Ethinyl, C₁-C₄-Alkyl, C₁-C₄-Alkoxy oder C₃-C₆-Cycloalkyl steht,
R⁸ für Wasserstoff, Amino, C₁-C₄-Alkyl, C₁-C₄-Alkylamino oder C₃-C₆-Cycloalkyl steht, und
R⁹ für Wasserstoff, Fluor, Chlor, Amino oder C₁-C₄-Alkyl steht,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassen, nachfolgend als Ausführungsbeispiele genannten Verbindungen und deren Salze, Solvate und Solvate der Salze, soweit es sich bei den von Formel (I) umfassen, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkoxycarbonyl, Alkylaminocarbonyl, Alkylaminosulfonyl und Alkylsulfonyl steht für einen linearen oder verzweigten Alkylrest mit in der Regel 1 bis 4, bevorzugt 1 oder 2 Kohlenstoffatomen, beispielhaft und vorzugsweise für Methyl, Ethyl, n-Propyl, Isopropyl und tert-Butyl.

Alkoxy steht beispielhaft und vorzugsweise für Methoxy, Ethoxy, n-Propoxy, Isopropoxy und tert-Butoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert-Butylamino, *N,N*-Dimethylamino, *N,N*-Diethylamino, *N*-Ethyl-*N-*methylamino, *N*-Methyl-*N*-n-propylamino, *N*-Isopropyl-*N*-n-propylamino und *N-tert*-Butyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl und tert-Butoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N*-Dimethylaminocarbonyl, *N,N*-Diethylaminocarbonyl, *N*-Ethyl-*N*-methylaminocarbonyl, *N-*Methyl-*N*-n-propylaminocarbonyl, *N*-iso-Propyl-*N*-n-propylaminocarbonyl und *N*-tert-Butyl-*N* methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonykest mit jeweils 1 bis 3 Kohlenstoffatomen pro Alkylsubstituent.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, iso-Propylaminosulfonyl, tert-Butylaminosulfonyl, *N,N-*Dimethylaminosulfonyl, *N,N*-Diethylaminosulfonyl, *N*-Ethyl-*N*-methylaminosulfonyl, *N*-Methyl-*N*-n-propylaminosulfonyl, *N*-iso-Propyl-*N*-n-propylaminosulfonyl und *N*-tert-Butyl-*N*-methylaminosulfonyl. C₁-C₃-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.

Alkylsulfonyl steht beispielhaft und vorzugsweise für Methylsulfonyl, Ethylsulfonyl, n-Propylsulfonyl, Isopropylsulfonyl und tert-Butylsulfonyl.

Cycloalkyl 1 steht für eine Cycloalkylgruppe mit in der Regel 3 bis 6 Kohlenstoffatomen, bevorzugt mit 3 bis 5 Kohlenstoffatomen, beispielhaft und vorzugsweise für Cyclopropyl, Cyclobutyl, Cyclopentyl und Cyclohexyl.

Heterocyclyl steht, wenn nicht weiter eingeschränkt, für einen monocyclischen Rest mit 4 bis 7 Ringatomen und bis zu 3, vorzugsweise bis zu 2 Heteroatomen und/oder Heterogruppen aus der Reihe N, O, S, SO, SO₂. Die eterocyclyl-Reste können gesättigt oder teilweise ungesättigt sein. Bevorzugt sind 5-gliedrige, monocyclische Heterocyclylreste mit bis zu zwei Heteroatomen aus der Reihe O, N und S, wie beispielhaft und vorzugsweise Tetrahydrofuranyl, Pyrrolidinyl, Pyrrolinyl, Isoxazolinyl und Pyrazolinyl.

Heteroaryl steht für einen aromatischen, monocyclischen Rest mit 5 Ringatomen und bis zu 4 Heteroatomen aus der Reihe S, O und N, beispielhaft und vorzugsweise für Thienyl, Furyl, Pyrrolyl, Thiazolyl, Oxazolyl, Isoxazolyl, Isothiazolyl, Imidazolyl und Pyrazolyl.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

In den Formeln der Gruppe, die für R⁴ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R⁴ gebunden ist.

In den Formeln der Gruppe, die für E stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das E gebunden ist.

Bevorzugt sind Verbindungen der Formel (I), in welcher
- E: für eine Gruppe der Formel 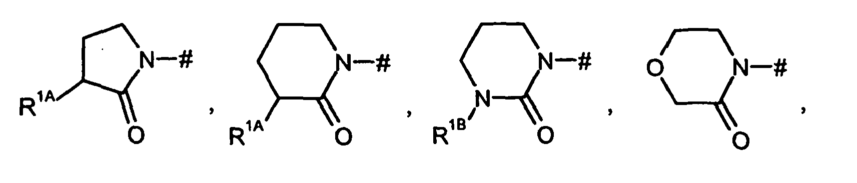 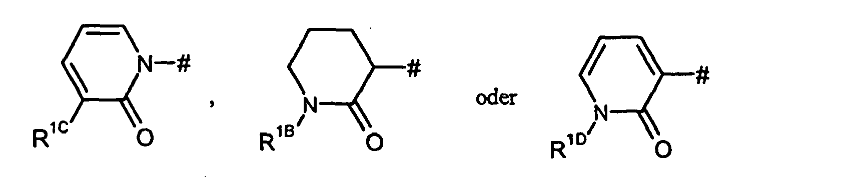 steht,
wobei
# die Anknüpfstelle an den Phenylring ist,
R^{1A} für Wasserstoff, Hydroxy, Hydroxymethyl, 2-Hydroxyethyl, Amino oder Methoxy steht,
R^{1B} für Wasserstoff, Hydroxy, Amino, Methyl oder Ethyl steht,
worin
Methyl substituiert sein kann mit einem Substituenten Pyrrolidin-1-yl,
und
Ethyl substituiert sein kann mit einem Substituenten, wobei der Substituent ausgewählt wird aus der Gruppe bestehend aus Hydroxy, Amino, Dimethylamino und Cyclopropylamino,
R^{1C} für Wasserstoff, Methyl oder Ethyl steht,
worin
Methyl substituiert sein kann mit einem Substituenten, wobei der Substituent ausgewählt wird aus der Gruppe bestehend aus Hydroxy und Pyrrolidin-1-yl, Ethyl substituiert sein kann mit einem Substituenten, wobei der Substituent ausgewählt wird aus der Gruppe bestehend aus Hydroxy, Amino und Cyclopropylamino, und
R^{1D} für Wasserstoff, Methyl oder Ethyl steht,
worin
Methyl substituiert sein kann mit einem Substituenten, wobei der Substituent ausgewählt wird aus der Gruppe bestehend aus Cyclopropylamino und Pyrrolidin-1-yl,
und
Ethyl substituiert sein kann mit einem Subsituenten, wobei der Substiuent ausgewählt wird aus der Gruppe bestehend aus Hydroxy, Amino und Cyclopropylamino,
- A: für ein 5-gliedriges Heteroaryl oder teilweise ungesättigtes 5-gliedriges Heterocyclyl steht,
wobei Heteroaryl und Heterocyclyl in 1 oder 2 Position an den Phenyl-Ring gebunden sind und Heteroaryl und Heterocyclyl selber eine 1,3-Verknüpfung mit dem Phenyl-Ring und der Carbonylaminomethyl-Gruppe aufweisen,
und
wobei Heteroaryl und Heterocyclyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl oder ein 5- oder 6-gliedriges Heteroaryl steht, wobei Phenyl und Heteroaryl substituiert sein können mit 1 bis 3 Substituenten, wobei die Substituenten unabhängig voneinander ausgewählt werden aus der Gruppe bestehend aus Halogen, Hydroxy, Amino, C₁-C₄-Alkyl, C₁-C₄-Alkoxy, C₁-C₄-Alkoxymethyl, C₁-C₄-Alkylamino, C₁-C₄-Alkylaminomethyl, Hydroxycarbonyl, Hydroxycarbonylmethyl, Aminocarbonyl, Aminocarbonylmethyl, C₁-C₄-Alkoxycarbonyl, C₁-C₄-Alkoxycarbonylmethyl, C₁-C₄-Alkylaminocarbonyl, C₁-C₄-Alkylaminocarbonylmethyl, Aminosulfonyl, C₁-C₄-Alkylaminosulfonyl und C₁-C₄-Alkylsulfonyl,
- R²: für Wasserstoff, Fluor, Chlor, Cyano, Hydroxy, C₁-C₄-Alkyl oder C₁-C₄-Alkoxy steht,
- R³: für Wasserstoff, Fluor, Chlor, Cyano, Hydroxy, C₁-C₄-Alkyl, C₁-C₄-Alkoxy, C₁-C₄-Alkoxymethyl, Cyclopropyl, Aminocarbonyl, C₁-C₄-Alkoxycarbonyl oder C₁-C₄-Alkylaminocarbonyl steht,
- R⁴: für eine Gruppe der Formel 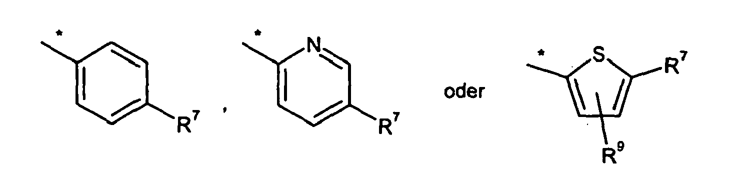 steht,
wobei
* die Anknüpfstelle an die Carbonylgruppe ist,
R⁷ für Fluor, Chlor, Ethinyl, Methyl oder Methoxy steht, und
R⁹ für Wasserstoff steht,
und ihre Salze, ihre Solvate und die Solvate ihrer Salze.

Bevorzugt sind auch Verbindungen der Formel (I), in welcher
- E: für eine Gruppe der Formel 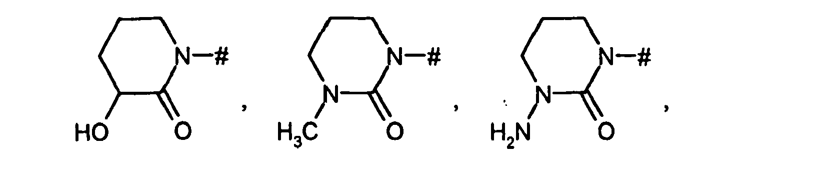 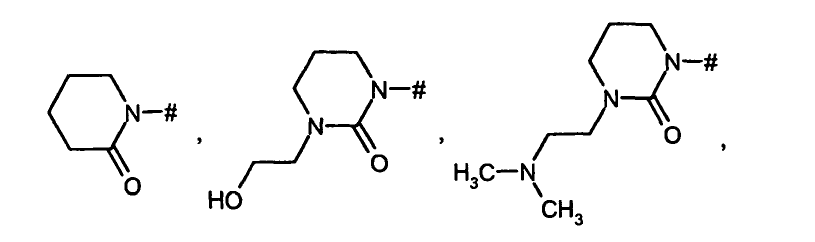 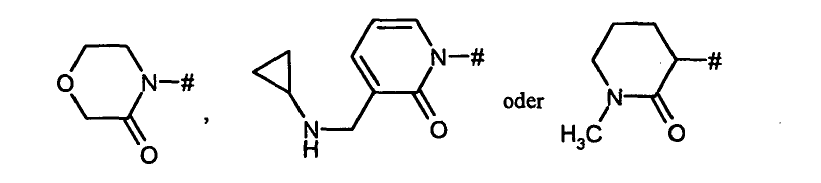 steht,
wobei
# die Anknüpfstelle an den Phenylring ist,
- A: für Pyrazolyl, Oxadiazolyl oder Isoxazolinyl steht,
wobei Pyrazolyl, Oxadiazolyl und Isoxazolinyl in 1 Position an den Phenyl-Ring gebunden sind und Pyrazolyl, Oxadiazolyl und Isoxazolinyl selber eine 1,3-Verknüpfung mit dem Phenyl-Ring und der Carbonylaminomethyl-Gruppe aufweisen,
und
wobei Pyrazolyl, Oxadiazolyl und Isoxazolinyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist und
wobei das Atom, an das R⁶ gebunden ist, ein Stickstoff- oder Kohlenstoffatom ist und
wobei
R⁶ für Phenyl, Pyrid-2-yl, Pyrid-3-yl, Pyrid-4-yl, 1,3-Oxazol-2-yl oder Pyrimidin-2-yl steht, wobei Phenyl, Pyrid-2-yl, Pyrid-3-yl, Pyrid-4-yl, 1,3-Oxazol-2-yl und Pyrimidin-2-yl substituiert sein können mit 1 bis 3 Substituenten, wobei die Substituenten unabhängig voneinander ausgewählt werden aus der Gruppe bestehend aus Halogen, Hydroxy, Amino, C₁-C₄-Alkyl, C₁-C₄-Alkoxy, C₁-C₄-Alkylamino, Hydroxycarbonyl, Aminocarbonyl, C₁-C₄-Alkoxycarbonyl, C₁-C₄-Alkylaminocarbonyl, Aminosulfonyl, C₁-C₄-Alkylaminosulfonyl und C₁-C₄-Alkylsulfonyl,
- R²: für Wasserstoff oder Fluor steht,
- R³: für Wasserstoff, Fluor, Chlor, Cyano, Methyl, Ethyl, n-Propyl, iso-Propyl, Methoxy, Ethoxy, Methoxymethyl oder Cyclopropyl steht,
- R⁴: für eine Gruppe der Formel 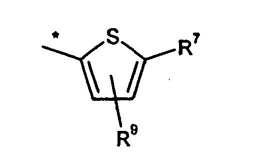 steht,
wobei
* die Anknüpfstelle an die Carbonylgruppe ist,
R⁷ für Fluor, Chlor oder Methyl steht, und
R⁹ für Wasserstoff steht,
und ihre Salze, ihre Solvate und die Solvate ihrer Salze.

Bevorzugt sind auch Verbindungen der Formel (I), in welcher
- E: für eine Gruppe der Formel 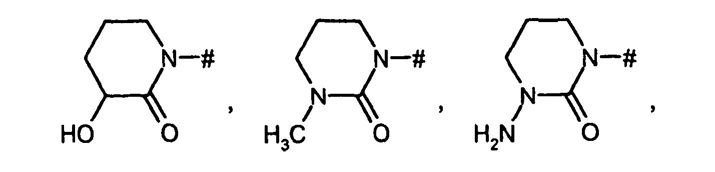 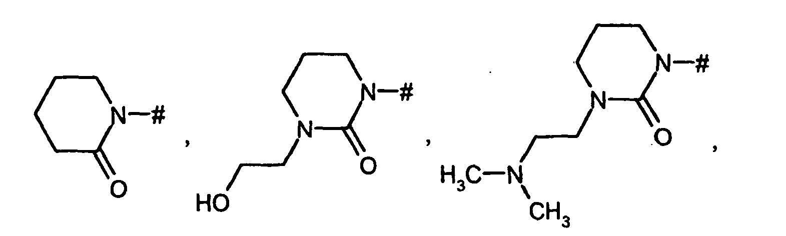 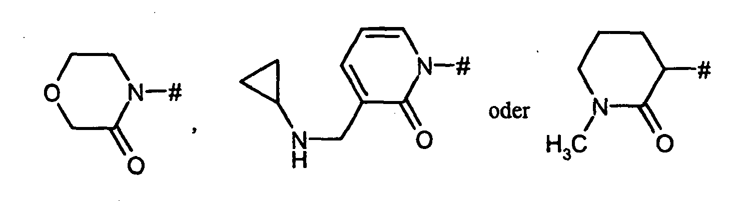 steht,
wobei
# die Anknüpfstelle an den Phenylring ist,
- A: für Pyrazolyl, Oxadiazolyl oder Isoxazolinyl steht,
wobei Pyrazolyl, Oxadiazolyl und Isoxazolinyl in 1 Position an den Phenyl-Ring gebunden sind und Pyrazolyl, Oxadiazolyl und Isoxazolinyl selber eine 1,3-Verknüpfung mit dem Phenyl-Ring und der Carbonylaminomethyl-Gruppe aufweisen,
und
wobei Pyrazolyl, Oxadiazolyl und Isoxazolinyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist und
wobei das Atom, an das R⁶ gebunden ist, ein Stickstoff- oder Kohlenstoffatom ist und
wobei
R⁶ für Phenyl, Pyrid-2-yl, Pyrid-3-yl oder Pyrid-4-yl steht,
wobei Phenyl, Pyrid-2-yl, Pyrid-3-yl und Pyrid-4-yl substituiert sein können mit einem Substituenten, wobei der Substituent ausgewählt wird aus der Gruppe bestehend aus Halogen, Hydroxy, Amino, C₁-C₄-Alkyl, C₁-C₄-Alkoxy, C₁-C₄-Alkylamino, Hydroxycarbonyl, Aminocarbonyl, C₁-C₄-Alkoxycarbonyl und C₁-C₄-Alkylaminocarbonyl,
- R²: für Wasserstoff oder Fluor steht,
- R³: für Wasserstoff, Fluor, Chlor, Methyl oder Methoxy steht,
- R⁴: für eine Gruppe der Formel 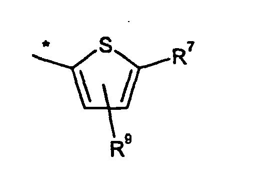 steht,
wobei
* die Anknüpfstelle an die Carbonylgruppe ist,
R⁷ für Chlor steht, und
R⁹ für Wasserstoff steht,
und ihre Salze, ihre Solvate und die Solvate ihrer Salze.

Bevorzugt sind auch Verbindungen der Formel (I), in welcher E für eine Gruppe der Formel 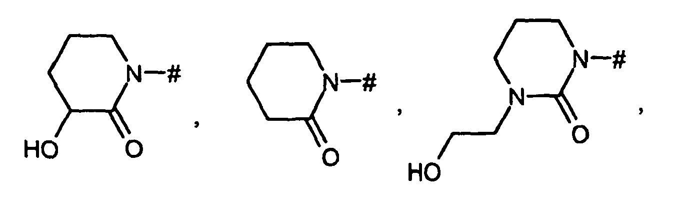 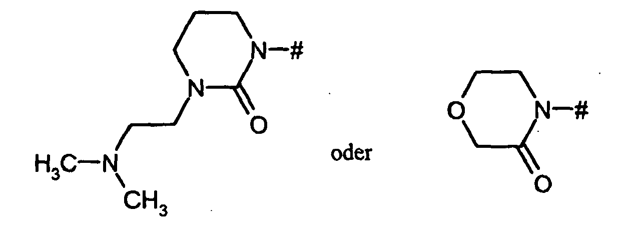 steht, wobei # die Anknüpfstelle an den Phenylring ist.

Bevorzugt sind auch Verbindungen der Formel (I), in welcher E für eine Gruppe der Formel 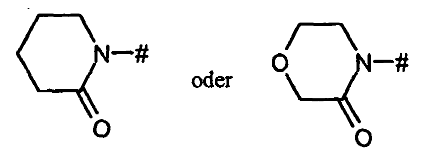 steht, wobei # die Anknüpfstelle an den Phenylring ist.

Bevorzugt sind auch Verbindungen der Formel (I), in welcher A für Pyrazolyl, Oxadiazolyl oder Isoxazolinyl steht.

Bevorzugt sind auch Verbindungen der Formel (I), in welcher A für Pyrazolyl steht.

Bevorzugt sind auch Verbindungen der Formel (I), in welcher R⁶ für Pyridyl, 4-Fluorphenyl oder 4-Methoxyphenyl steht.

Bevorzugt sind auch Verbindungen der Formel (I), in welcher R² für Wasserstoff oder Fluor steht.

Bevorzugt sind auch Verbindungen der Formel (I), in welcher R³ für Wasserstoff, Fluor, Chlor, Methyl oder Methoxy steht.

Bevorzugt sind auch Verbindungen der Formel (I), in welcher R³ für Wasserstoff steht.

Bevorzugt sind auch Verbindungen der Formel (I), in welcher R² und R³ für Wasserstoff stehen.

Bevorzugt sind auch Verbindungen der Formel (I), in welcher R² für Wasserstoff und R³ für Fluor steht.

Bevorzugt sind auch Verbindungen der Formel (I), in welcher R⁴ für eine Gruppe der Formel 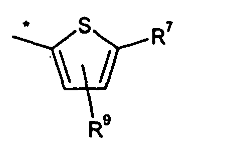 steht, wobei * die Anknüpfstelle an die Carbonylgruppe ist, R⁷ für Chlor steht und R⁹ für Wasserstoff steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei Verbindungen der Formel 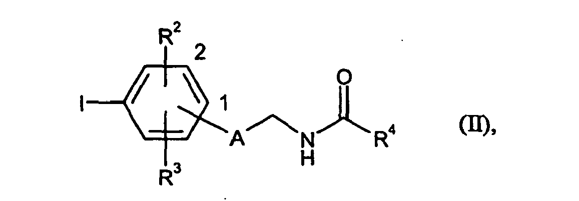 in welcher A, R², R³ und R⁴ die oben angegebene Bedeutung haben,
mit Verbindungen der Formel

E-H (III),

in welcher E die oben angegebene Bedeutung hat,
umgesetzt werden.

Die Umsetzung erfolgt für Verbindungen der Formel (III), in denen der in Formel (III) gezeigte Wasserstoff über ein Stickstoffatom an E angebunden ist, im Allgemeinen in inerten Lösungsmitteln unter Zugabe eines Kupfer(I)-Salzes, einer Base und eines Diamin-Liganden, bevorzugt in einem Temperaturbereich von 60°C bis zum Rückfluss des Lösungsmittels bei Normaldruck.

Inerte Lösungsmittel sind beispielsweise aprotische Lösungsmittel wie Toluol, Dioxan, Tetrahydrofuran oder Dimethylformamid, bevorzugt ist Dioxan.

Kupfer(I)-Salze sind beispielsweise Kupfer(I)-iodid, Kupfer(1)-chlorid oder Kupfer(I)-oxid, bevorzugt ist Kupfer(I)-iodid.

Basen sind beispielsweise Kaliumphosphat, Kaliumcarbonat oder Cäsiumcarbonat, bevorzugt ist Kaliumphosphat.

Diamin-Liganden sind beispielsweise 1,2-Diamine wie *N,N*'-Dimethylethylendiamin.

Für Verbindungen der Formel (III), in denen der in Formel (III) gezeigte Wasserstoff über ein Kohlenstoffatom an E angebunden ist, erfolgt die Umsetzung im Allgemeinen in inerten Lösemitteln zunächst unter Zugabe einer starken Base, dann Zusatz eines Zinksalzes und abschließendem Zusatz einer Verbindung der Formel (II) und eines Palladium-Komplexes. Die ersten beiden Teilschritte, die Reaktion mit der starken Base und die Umsetzung mit dem Zinksalz, erfolgen bevorzugt in einem Temperaturbereich von -30 bis 0°C; der letzte Teilschritt bevorzugt bei Raumtemperatur bis zum Siedepunkt des Lösemittels.

Inerte Lösemittel sind beispielsweise Ether wie Tetrahydrofuran, Dioxan oder 1,2-Dimethoxyethan; gegebenenfalls im Gemisch mit Kohlenwasserstoffen wie beispielsweise Hexan. Bevorzugt ist Tetrahydrofuran.

Starke Basen sind beispielsweise sec-Butyllithium, tert-Butyllithium, Lithiumdiisopropylamid oder Lithiumhexamethyldisilazid. Bevorzugt ist sec-Butyllithium.

Das bevorzugte Zinksalz ist Zinkchlorid.

Palladium-Komplexe werden *in situ* aus Palladiumverbindungen und Liganden gebildet. Als Palladiumverbindungen eignen sich zum Beispiel Palladium(II)-acetat, Palladium(II)-chlorid, Bis(triphenylphosphin)palladium(II)-chlorid, Tetrakis(triphenylphosphin)palladium(0), Bis(dibenzylidenacton)palladium(0). Bevorzugt ist Bis(dibenzylidenacton)palladium(0). Als Liganden eignen sich zum Beispiel 2-Dicyclohexylphosphino-2'-(*N,N*-dimethylamino)biphenyl, Binaphthyl oder N-heterocyclische Carbenliganden. Bevorzugt ist 2-Dicyclohexylphosphino-2'-(*N,N-*dimethylamino)biphenyl.

Die Verbindungen der Formel (III) sind bekannt oder lassen sich nach bekannten Verfahren aus den entsprechenden Ausgangsverbindungen synthetisieren.

Die Verbindungen der Formel (II) sind bekannt oder können hergestellt werden, indem Verbindungen der Formel 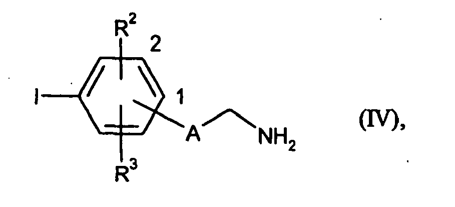 in welcher A, R² und R³ die oben angegebene Bedeutung haben,
mit Verbindungen der Formel 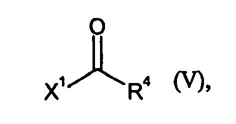 in welcher R⁴ die oben angegebene Bedeutung hat, und
- X¹: für Halogen, bevorzugt Brom oder Chlor, oder Hydroxy steht,
umgesetzt werden.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Pyridin, Tetrahydrofuran oder Methylenchlorid.

Basen sind beispielsweise Triethylamin, Diisopropylethylamin oder N-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N*,'-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O-*(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (IV) sind bekannt oder lassen sich nach dem Fachmann bekannten Verfahren zum Aufbau des Heterozyklus A aus den entsprechenden Ausgangsverbindungen synthetisieren.

Die Verbindungen der Formel (V) sind bekannt oder lassen sich nach bekannten Verfahren aus den entsprechenden Ausgangsverbindungen synthetisieren.

Der Stickstoff des Amides in Verbindungen der Formeln (II) und (IV) kann gegebenenfalls während der Umsetzung mit einer dem Fachmann bekannten Schutzgruppe geschützt sein, bevorzugt ist eine 2,4-Dimethoxybenzyl-Gruppe, die unter den Bedingungen der letzten Stufe der Synthese der Verbindungen der Formel (I) abgespalten wird.

Die Herstellung der erfindungsgemäßen Verbindungen kann durch das folgende Syntheseschemata veranschaulicht werden:

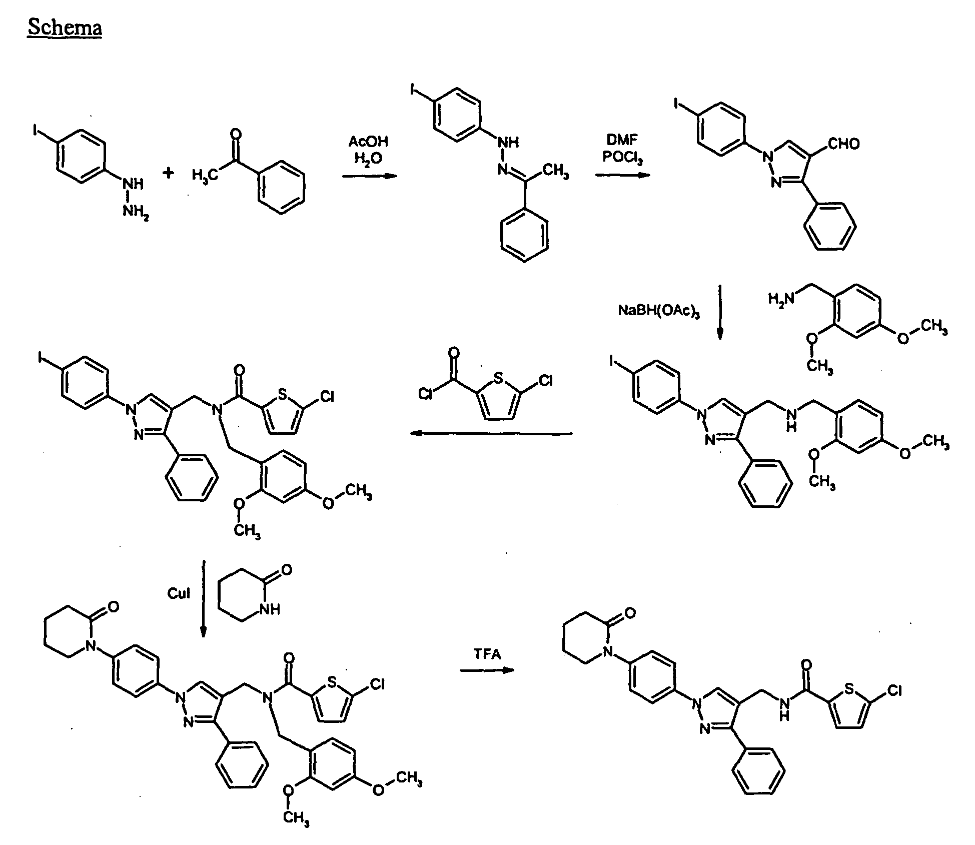

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken.

Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrornbosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodi/Vatatoren, insbesondere AcE-(Angiotensin-Convening-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- DC: Dünnschicht-Chromatographie
- DCI: direkte chemische Ionisation (bei MS)
- DMF: *N,N*-Dimethylformamid
- DMSO: Dimethylsulfoxid
- d: Tag(e)
- d. Th.: der Theorie (bei Ausbeute)
- ee: Enantiomerenüberschuss
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- RP: reverse phase (bei HPLC)
- RT: Raumtemperatur
- Rₜ: Retentionszeit (bei HPLC)
- TBTU: *O*-(Benzotriazol-1-yl)-*N,N,N',N'*-tetramethyluronium-tetrafluoroborat
- THF: Tetrahydrofuran

### LC-MS- und HPLC-Methoden

Methode 1: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 2: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

Methode 3: Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 4: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 5: Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208-400 nm.

Methode 6: Säule: GROM-SIL 120 ODS-4 HE, 10 µM, 250 mm x 30 mm; Laufmittel und Gradientenprogramm: Acetonitril/0.1% wässrige Ameisensäure 10:90 (0-3 min), Acetonitril/0.1% wässrige Ameisensäure 10:90 → 95:5 (3-27 min), Acetonitril/0.1% wässrige Ameisensäure 95:5 (27-34 min), Acetonitril/0.1% wässrige Ameisensäure 10:90 (34-38 min); Fluss: 50 ml/min; Temperatur: 22°C; UV-Detektion: 254 nm.

### Ausgangsverbindungen

### Beispiel 1A

### Acetophenon-(4-jodphenyl)hydrazon

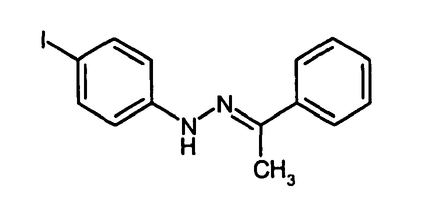

Eine Lösung von 2.0 g (8.546 mmol) 4-Jodphenylhydrazin in 30 ml 50%-iger Essigsäure wird mit einer Lösung von 1.54 g (12.82 mmol) Acetophenon in 10 ml des gleichen Lösemittels versetzt. Es wird bei Raumtemperatur gerührt, wobei ein Niederschlag ausfällt. Nach 30 Minuten wird der Niederschlag abfiltriert und nacheinander mit Wasser und Cyclohexan gut gewaschen. Der Rückstand wird im Hochvakuum getrocknet. Es werden 1.95 g (68% d. Th.) der Titelverbindung erhalten.

¹H-NMR (400 MHz, DMSO-d₆, δ/*ppm*): 7.78 (d, 2H), 7.51 (d, 2H), 7.38 (dd, 2H), 7.30 (dd, 1H), 7.07 (d, 2H), 2.25 (s, 3H).

HPLC (Methode 4): Rₜ = 3.22 min.

MS (ESIpos, *m*/*z*): 337 (M+H)⁺.

### Beispiel 2A

### 1-(4-Jodphenyl)-3-phenyl-1H-pyrazol-4-carbaldehyd

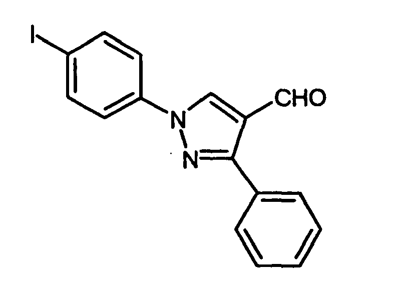

Bei 0°C werden 1.08 ml (11.58 mmol) Phosphorylchlorid (POCl₃) langsam zu 10 ml wasserfreiem *NN*-Dimethylformamid zugetropft. Nach 30 Minuten bei 0°C wird eine Lösung von 1.95 g (5.792 mmol) des Produktes aus Beispiel 1A in 10 ml *N,N*-Dimethylformamid zugetropft und das Reaktionsgemisch eine weitere Stunde bei 0°C gerührt. Dann läßt man auf Raumtemperatur erwärmen, rührt eine weitere Stunde, bevor man auf 60°C erwärmt. Das Reaktionsgemisch wird 15 Stunden bei dieser Temperatur gerührt. Anschließend läßt man auf Raumtemperatur abkühlen, versetzt mit 80 ml gesättigter Natriumhydrogencarbonat-Lösung und extrahiert mit Ethylacetat. Der organische Extrakt wird nacheinander mit Wasser und gesättigter Kochsalz-Lösung gewaschen. Nach Trocknen über wasserfreiem Natriumsulfat wird filtriert und das Lösemittel am Rotationsverdampfer entfernt. Der erhaltene Rückstand wird mit Diisopropylether verrieben. Der Feststoff wird abgesaugt, mit Diisopropylether gewaschen und im Hochvakuum getrocknet. Es werden 1.34 g (62% d. Th.) der Titelverbindung erhalten.

¹H-NMR (400 MHz, DMSO-d₆, δ/*ppm*): 9.98 (s, 1H), 9.36 (s, 1H), 7.95-7.90 (m, 4H), 7.82 (d, 2H), 7.53-7.48 (m, 3H).

HPLC (Methode 4): Rₜ = 3.08 min.

MS (ESIpos, *m*/*z*): 375 (M+H)⁺.

### Beispiel 3A

### 1-(2,4-Dimethoxyphenyl)-N-{[1-(4 jodphenyl)-3-phenyl-1Hpyrazol-4-yl]methyl}methanamin

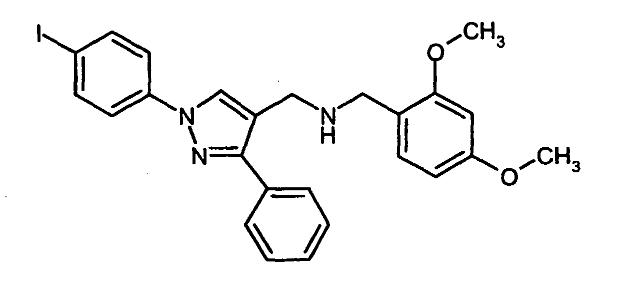

1.34 g (3.581 mmol) des Produktes aus Beispiel 2A und 538 µl (3.581 mmol) 2,4-Dimethoxybenzylamin werden in 40 ml Dichlorethan gelöst und eine Stunde bei Raumtemperatur gerührt. Dann werden 1.52 g (7.162 mmol) Natriumtriacetoxyborhydrid und 820 µl (14.33 mmol) Eisessig zugefügt. Das Reaktionsgemisch wird 15 Stunden bei Raumtemperatur gerührt. Anschließend wird gesättigte Natriumhydrogencarbonat-Lösung zugesetzt und das Produkt mit Dichlormethan extrahiert. Der organische Extrakt wird mit Wasser gewaschen und über wasserfreiem Natriumsulfat getrocknet. Nach Filtration wird das Lösemittel am Rotationsverdampfer entfernt. Das Rohprodukt wird im Hochvakuum getrocknet und ohne weitere Reinigung in der nächsten Reaktion eingesetzt. Es werden 1.89 g der Titelverbindung erhalten.

HPLC (Methode 5): Rₜ = 2.10 min (60%).

MS (ESIpos, *m*/*z*): 526 (M+H)⁺.

### Beispiel 4A

### 5-Chlor-N-(2,4-dimethoxybenzyl)-N-{[1-(4-jodphenyl)-3-phenyl-1H-pyrazol-4-yl]methyl}-thiophen-2-carbonsäureamid

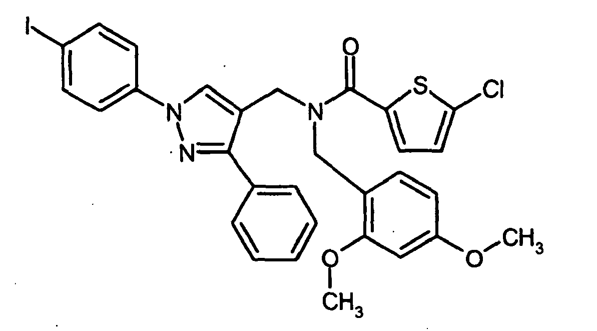

Eine Lösung von 1.89 g (3.597 mmol) des Produktes aus Beispiel 3A und 1.25 ml Diisopropylethylamin (Hünig-Base) in 40 ml wasserfreiem Tetrahydrofuran werden mit einer Lösung von 651 mg (3.597 mmol) 5-Chlorthiophen-2-carbonsäurechlorid in 10 ml wasserfreiem Tetrahydrofuran versetzt. Das Reaktionsgemisch wird 15 Stunden bei Raumtemperatur gerührt. Anschließend wird das Lösemittel am Rotationsverdampfer entfernt, der Rückstand in Dichlormethan aufgenommen und nacheinander mit gesättigter Natriumhydrogencarbonat-Lösung und Wasser gewaschen. Nach Trocknen über wasserfreiem Natriumsulfat wird filtriert, eingedampft und der Rückstand mittels präparativer HPLC (Methode 7) gereinigt. Es werden 1.05 g (43% d. Th.) der Titelverbindung erhalten.

¹H-NMR (500 MHz, DMSO-d₆, δ/*ppm*): 8.53 (breit, 1H), 7.85 (d, 2H), 7.76 (d, 2H), 7.61 (d, 2H), 7.43-7.38 (m, 3H), 7.15 (breit, 1H), 7.08 (d, 1H), 7.01 (breit, 1H), 6.48-6.43 (m, 2H), 4.70 (breit, 2H), 4.58 (s, breit, 2H), 3.71 (s, 3H), 3.57 (breit, 3H).

HPLC (Methode 2): Rₜ = 6.47 min.

MS (ESIpos, *m*/*z*): 670/672 (³⁵Cl/³⁷Cl) (M+H)⁺.

### Beispiel 5A

### 5-Chlor-N-(2,4-dimethoxybenzyl)-N-({(1-[4-(3-oxomorpholin-4-yl)phenyl]-3-phenyl-1H-pyraol-4-yl}methyl)thiophen-2-carbonsäureamid

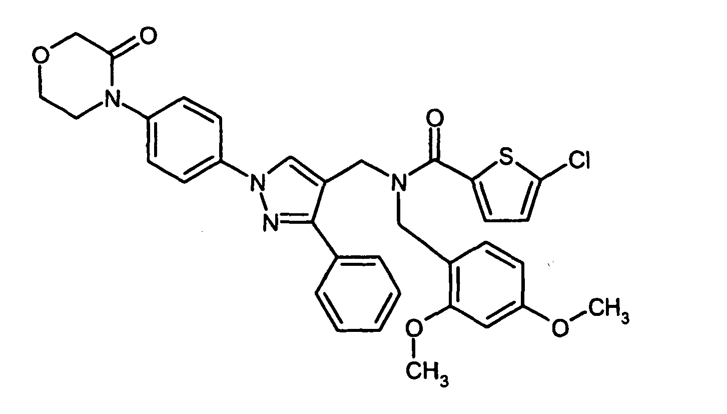

136 mg (0.203 mmol) des Produktes aus Beispiel 4A werden in 10 ml wasserfreiem Dioxan gelöst und nacheinander mit 20.5 mg (0.203 mmol) Morpholinon, 8 mg (0.041 mmol) Kupfer(I) jodid, 86 mg (0.406 mmol) Kaliumphosphat und 6.5 µl (0.061 mmol) *N,N'*-Dimethylethylendiamin versetzt. Die Rückflußapparatur wird durch wiederholtes Anlegen eines leichten Vakuums und Begasen mit Argon-inertisiert. Das-Reaktionsgemisch wird zwei Tage zum Rückfluß erhitzt. Nach dieser Zeit läßt man auf RT abkühlen. Es wird mit Wasser versetzt und mit Ethylacetat extrahiert. Der organische Extrakt wird nacheinander mit Wasser und gesättigter Kochsalz-Lösung gewaschen. Es wird über wasserfreiem Magnesiumsulfat getrocknet, filtriert und das Filtrat im Vakuum vom Lösemittel befreit. Der Rückstand wird mittels präparativer HPLC (Methode 7) gereinigt. Es werden 67 mg des Eduktes wiedergewonnen und 40 mg (60% d. Th., bezogen auf 51 % Umsatz) der Titelverbindung erhalten.

¹H-NMR (400 MHz, DMSO-d₆, δ/*ppm*): 8.53 (s, breit, 1H), 7.97 (d, 2H), 7.62 (d, 2H), 7.56 (d, 2H), 7.44-7.37 (m, 3H), 7.17 (breit, 1H), 7.10 (d, 1H), 7.02 (breit, 1H), 6.50-6.44 (m, 2H), 4.73 (breit, 2H), 4.59 (breit, 2H), 4.23 (s, 2H), 4.01 (t, 2H), 3.80 (t, 2H), 3.72 (s, 3H), 3.57 (s, breit, 3H).

HPLC (Methode 3): Rₜ = 2.69 min.

MS (ESIpos, *m*/*z*): 643/645 (³⁵Cl/³⁷Cl) (M+H)⁺.

### Beispiel 6A

### 4-Jod-3-methylphenylhydrazin

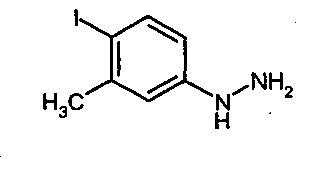

Eine Suspension von 1.165 g (5.0 mmol) 4-Jod-3-methylanilin in 5 ml konzentrieter Salzsäure wird bei -10°C tropfenweise mit einer Lösung von 345 mg (5.0 mmol) Natriumnitrit in 5 ml Wasser versetzt. Es wird 45 Minuten bei ca. -5°C gerührt, bevor bei derselben Temperatur eine Lösung von 5.416 g (24.0 mmol) Zinn(II)-chlorid-Dihydrat in 4.5 ml konzentrierter Salzsäure hinzugetropft wird. Anschliessend wird zehn Minuten bei 0°C nachgerührt. Anschliessend wird durch Zugabe von festem Natriumhydroxid das Reaktionsgemisch auf einen pH-Wert von ca. 14 gebracht. Der entstandene Niederschlag wird abfiltriert. Das Filtrat wird mit Dichlormethan extrahiert. Der Extrakt wird mit Wasser gewaschen, über wasserfreiem Natriumsulfat getrocknet, filtriert und eingeengt. Das Rohprodukt wird über Kieselgel mit Dichlormethan/Methanol 40: als Laufmittel gereinigt. Es werden 555 mg (38% d. Th., bezogen auf 85% Reinheit) der Titelverbindung erhalten.

¹H-NMR (400 MHz, DMSO-*d*₆, δ/*ppm*): 7.42 (d, 1H), 6.79 (breit, 1H), 6.77 (d, 1H), 6.39 (dd, 1H), 3.93 (breit, 2H), 2.23 (s, 3H).

HPLC (Methode 1): Rₜ = 3.53 min.

MS (EI, m/z): 248 (M)⁺.

### Beispiel 7A

### Acetophenon-(4-jod-3-methylphenyl)hydrazon

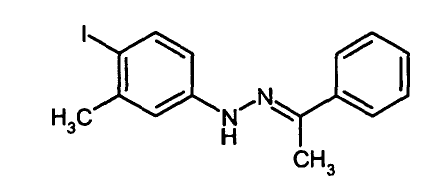

Analog zu dem unter Beispiel 1A beschriebenen Verfahren werden aus 992 mg (4.0 mmol) der Verbindung aus Beispiel 6A und 721 mg (6.0 mmol) Acetophenon 1.405 g (100% d. Th.) der Titelverbindung erhalten.

MS (DCI, NH₃, *m*/*z*): 351 (M+H)⁺.

### Beispiel 8A

### 1-(4-Jod-3-methylphenyl)-3-phenyl-1H-pyrazol-4-carbaldchyd

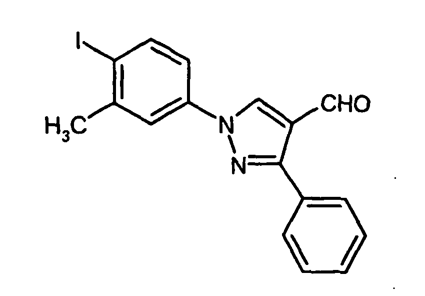

Analog zu dem unter Beispiel 2A beschriebenen Verfahren werden 700 mg (1.999 mmol) der Verbindung aus Beispiel 7A zu 695 mg der Titelverbindung erhalten (59% d. Th., bezogen auf 66% Reinheit).

¹H-NMR (400 MHz, DMSO-d₆, δ/*ppm*): 9.98 (s, 1H), 9.35 (s, 1H), 8.03 (d, 1H), 8.01 (d, 1H), 7.93-7.91 (m, 2H), 7.61 (dd, 1H), 7.53-7.50 (m, 3H), 2.47 (s, 3H).

HPLC (Methode 5): Rₜ = 3.15 min.

MS (ESIpos, *m*/*z*): 389 (M+H)⁺.

### Beispiel 9A

### 1-(2,4-Dimethoxyphenyl)-N-{[1-(4-jod-3-methylphenyl)-3-phenyl-1H-pyrazol-4-yl]methyl}methanamin

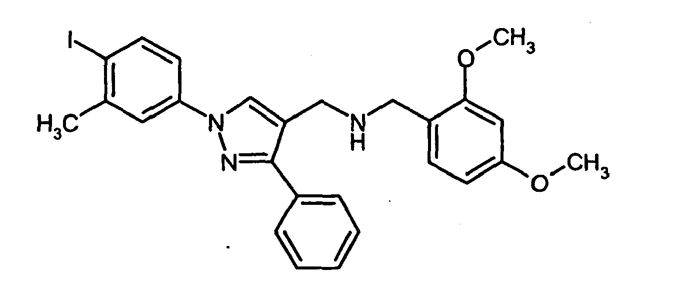

Analog zu dem in Beispiel 3A beschriebenen Verfahren werden aus 680 mg (1.752 mmol) der Verbindung aus Beispiel 8A 288 mg der Titelverbindung erhalten (27% d. Th., bezogen auf 90% Reinheit).

¹H-NMR (400 MHz, DMSO-d₆, δ/*ppm*): 8.49 (s, 1H), 7.92 (d, 1H), 7.92 (breit, 1H), 7.83 (d, 2H), 7.49 (dd, 1H), 7.43 (dd, 2H), 7.37 (dd, 1H), 7.22 (d, 1H), 6.53 (d, 1H), 6.47 (dd, 1H), 3.75 (s, 3H), 3.74 (s, 3H), 3.72-3.70 (m, 4H), 2.54 (s, 3H).

HPLC (Methode 2): Rₜ = 5.00 min.

MS (ESIpos, *m*/*z*): 540 (M+H)⁺.

### Beispiel 10A

### 5-Chlor-N-(2,4-dimethoxybenzyl)-N-{[1-(4-jod-3-methylphenyl)-3-phenyl-1H-pyrazol-4-yl]methyl}thiophen-2-carbonsäureamid

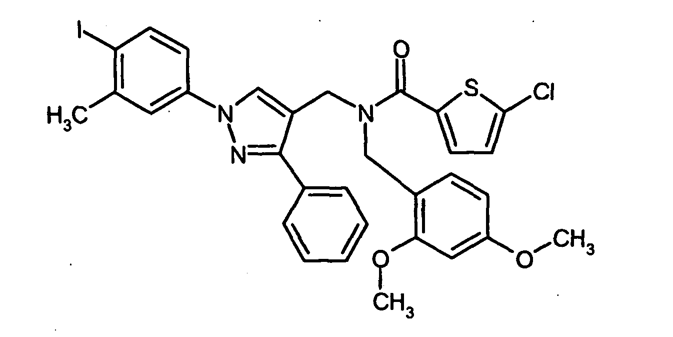

Analog zu dem in Beispiel 4A beschriebenen Verfahren werden aus 284 mg (0.526 mmol) der Verbindung aus Beispiel 9A 315 mg (84% d. Th.) der Titelverbindung erhalten.

¹H-NMR (400 MHz, DMSO-d₆, δ/*ppm*): 8.50 (breit, 1H), 7.93-7.91 (m, 2H), 7.61 (d, 2H), 7.53 (dd, 1H), 7.44-7.38 (m, 3H), 7.17 (breit, 1H), 7.08 (d, 1H), 7.02 (breit, 1H), 6.48 (breit, 1H), 6.45 (dd, 1H), 4.72 (breit, 2H), 4.58 (breit, 2H), 3.71 (s, 3H), 3.56 (breit, 3H), 2.47 (s, 3H).

HPLC (Methode 2): Rₜ = 6.52 min.

MS (ESIpos, *m*/*z*): 684/686 (³⁵Cl/³⁷Cl) (M+H)⁺.

### Beispiel 11A

### 5-Chlor-N-(2,4-dimethoxybenzyl)-N-({1-[3-methyl-4-(3-oxomorpholin-4-yl)phenyl]-3-phenyl-1H-pyrazol-4-yl} methyl)thiophen-2-carbonsäureamid

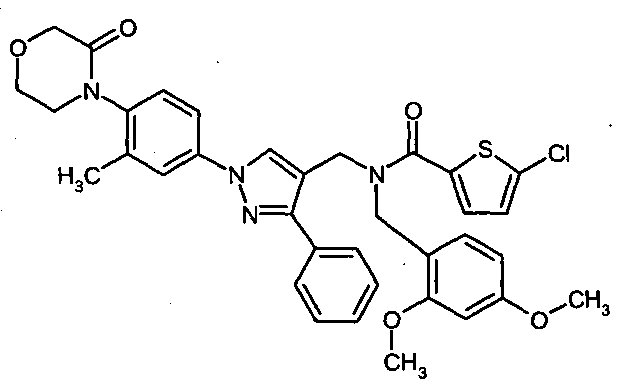

Analog zu dem in Beispiel 5A beschriebenen Verfahren werden aus 150 mg (0.219 mmol) der Verbindung aus Beispiel 10A und 33.3 mg (0.329 mmol) Morpholinon 93 mg (64% d. Th.) der Titelverbindung erhalten.

¹H-NMR (400 MHz, DMSO-d₆, δ/*ppm*): 8.48 (breit, 1H), 7.87 (d, 1H), 7.80 (dd, 2H), 7.61 (d, 2H), 7.45-7.37 (m, 4H), 7.16 (breit, 1H), 7.08 (d, 1H), 7.02 (breit, 1H), 6.48 (breit, 1H), 6.44 (dd, 1H), 4.72 (breit, 2H), 4.59 (breit, 2H), 4.27-4.18 (m, 2H), 4.03-3.98 (m, 2H), 3.71 (s, 3H), 3.59-3.47 (m, 2H), 3.56 (s, 3H), 2.23 (s, 3H).

HPLC (Methode 1): Rₜ = 5.15 min.

MS (ESIpos, *m*/*z*): 657/659 (³⁵Cl/³⁷Cl) (M+H)⁺.

### Beispiele 12A

### Methyl-3-pyridylketon-(4-jodphenyl)hydrazon

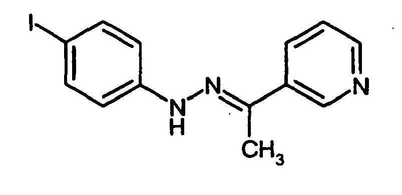

Analog zu dem in Beispiel 1A beschriebenen Verfahren werden aus 5.0 g (21.36 mmol) 4-Jodphenylhydrazin und 3-Acetylpyridin 5.15 g (71% d. Th.) der Titelverbindung erhalten.

¹H-NMR (500 MHz, DMSO-d₆, δ/*ppm*): 9.61 (s, 1H), 8.97 (s, 1H), 8.48 (d, 1H), 8.12 (d, 1H), 7.52 (d, 2H), 7.40 (dd, 1H), 7.11 (d, 2H), 2.28 (s, 3H).

HPLC (Methode 1): Rₜ = 4.09 min.

MS (DCI, NH₃, *m*/*z*): 338 (M+H)⁺.

### Beispiel 13A

### 1-(4-Jodphenyl)-3-pyridin-3-yl-1H-pyrazol-4-carbaldehyd

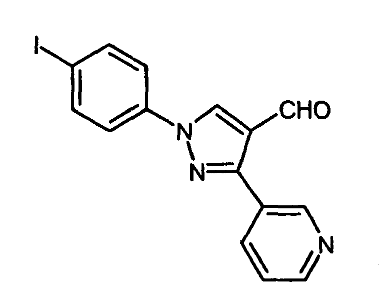

Analog zu dem in Beispiel 2A beschriebenen Verfahren werden aus 5.1 g (15.12 mmol) der Verbindung aus Beispiel 12A 4.33 g (76% d. Th.) der Titelverbindung erhalten.

¹H-NMR (400 MHz, DMSO-d₆, δ/*ppm*): 9.99 (s, 1H), 9.43 (s, 1H), 9.10 (d, 1H), 8.68 (dd, 1H), 8.32 (d, 1H), 7.95 (d, 2H), 7.83 (d, 2H), 7.56 (dd, 1H).

HPLC (Methode 1): Rₜ = 3.99 min.

MS (DCI, NH₃, *m*/*z*): 376 (M+H)⁺, 393 (M+NH₄)⁺.

### Beispiel 14A

### 1-(2,4-Dimethoxyphenyl)-N-{[1-(4-jodphenyl)-3-pyridin-3-yl-1H-pyrazol-4-yl]methyl)-methanamin

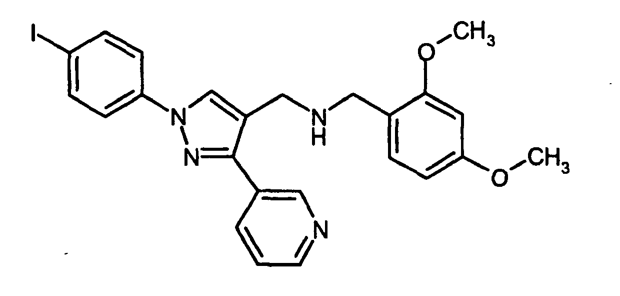

Analog zu dem unter Beispiel 3A beschriebenen Verfahren werden aus 4.33 g (11.54 mmol) der Verbindung aus Beispiel 13A 6.07 g (99% d. Th.) der Titelverbindung erhalten, die ohne weitere Aufreinigung in der nächsten Synthesestufe eingesetzt wird.

HPLC (Methode 5): Rₜ = 1.75 min (60%).

MS (ESIpos, *m*/*z*): 527 (M+H)⁺.

### Beispiel 15A

### 5-Chlor-N-(2,4-dimethoxybenzyl)-N-{[1-(4-jodphenyl)-3-pyridin-3-yl-1H-pyrazol-4-yl]methyl}-thiophen-2-carbonsäureamid

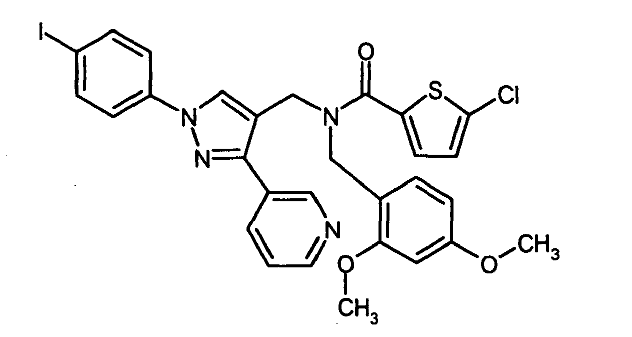

Analog zu dem unter Beispiel 4A beschriebenen Verfahren werden 6.07 g (11.54 mmol) der Verbindung aus Beispiel 14A zu 2.57 g (33% d. Th.) der Titelverbindung umgesetzt.

¹H-NMR (400 MHz, DMSO-d₆, δ/*ppm*): 8.81 (d, 1H), 8.58-8.53 (m, 2H), 7.98 (dd, 1H), 7.87 (d, 2H), 7.77 (d, 2H), 7.43 (dd, 1H), 7.17 (breit, 1H), 7.09 (d, 1H), 7.00 (breit, 1H), 6.46 (breit, 1H), 6.42 (dd, 1H), 4.70 (breit, 2H), 4.58 (breit, 2H), 3.71 (s, 3H), 3.57 (breit, 3H).

HPLC (Methode 2): Rₜ = 4.95 min.

MS (ESIpos, *m*/*z*): 671/673 (³⁵Cl/³⁷Cl) (M+H)⁺.

### Beispiel 16A

### 5-Chlor-N-(2,4-dimethoxybenzyl)-N-({1-[4-(3-oxomorpholin-4-yl)phenyl]-3-pyridin-3-yl-1H-pyrazol-4-yl}methyl)thiophen-2-carbonsäureamid

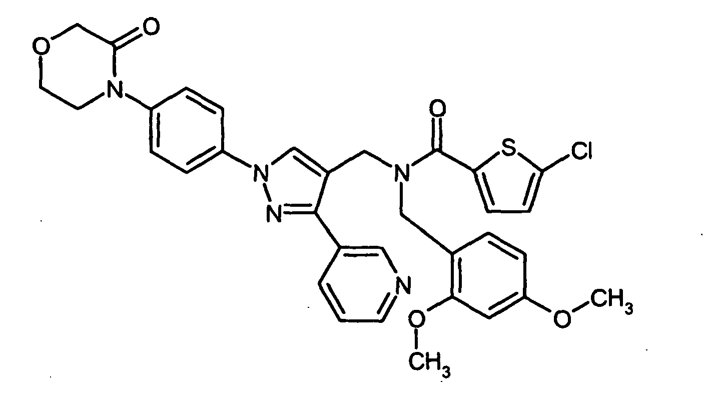

Analog zu dem unter Beispiel 5A beschriebenen Verfahren werden 145 mg (0.216 mmol) der Verbindung aus Beispiel 15A zu 117 mg (81% d. Th.) der Titelverbindung umgesetzt.

¹H-NMR (400 MHz, DMSO-d₆, δ/*ppm*): 8.81 (d, 1H), 8.57 (dd, 1H), 8.53 (breit, 1H), 7.99 (dd, 1H), 7.95 (d, 2H), 7.57 (d, 2H), 7.45 (dd, 1H), 7.17 (breit, 1H), 7.08 (d, 1H), 6.99 (breit, 1H), 6.47 (breit, 1H), 6.43 (dd, 1H), 4.72 (breit, 2H), 4.59 (breit, 2H), 4.23 (s, 2H), 4.01 (t, 2H), 3.79 (t, 2H), 3.72 (s, 3H), 3.58 (s, breit, 3H).

HPLC (Methode 2): Rₜ = 4.26 min.

MS (ESIpos, *m*/*z*): 644/646 (³⁵Cl/³⁷Cl) (M+H)⁺.

### Beispiel 17A

### 5-Chlor-N-(2,4-dimethoxybenzyl)-N-({1-[4-(2-oxopiperidin-1-yl)phenyl]-3-pyridin-3-yl-1H-pyrazol-4-yl}methyl)thiophen-2-carbonsäureamid

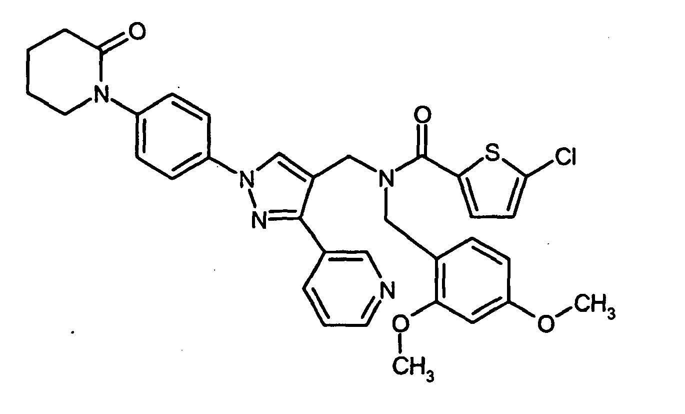

Analog zu dem unter Beispiel 5A beschriebenen Verfahren werden 177 mg (0.264 mmol) der Verbindung aus Beispiel 15A und Piperidin-2-on zu 70 mg (40% d. Th.) der Titelverbindung umgesetzt.

¹H-NMR (500 MHz, DMSO-d₆, δ/*ppm*): 8.83 (breit, 1H), 8.58 (breit, 1H), 8.53 (breit, 1H), 8.00 (d, 1H), 7.92 (d, 2H), 7.47-7.41 (m, 3H), 7.17 (breit, 1H), 7.08 (d, 1H), 6.99 (breit, 1H), 6.47 (breit, 1H), 6.43 (dd, 1H), 4.72 (breit, 2H), 4.59 (breit, 2H), 3.71 (s, 3H), 3.64 (t, 2H), 3.57 (s, breit, 3H), 2.42 (t, 2H), 1.91-1.83 (m, 4H).

HPLC (Methode 2): Rₜ = 4.34 min.

MS (DCI, NH₃, *m*/*z*): 642/644 (³⁵Cl³⁷Cl) (M+H)⁺.

### Beispiel 18A

### Methyl-4-pyridylketon-(4-jodphenyl)hydrazon

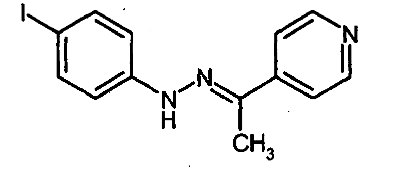

Analog zu dem in Beispiel 1A beschriebenen Verfahren werden aus 5.0 g (21.36 mmol) 4-Jodphenylhydrazin und 4-Acetylpyridin 6.67 g (92% d. Th.) der Titelverbindung erhalten.

¹H-NMR (500 MHz, DMSO-d₆, δ/*ppm*): 9.78 (s, 1H), 8.54 (d, 2H), 7.70 (d, 2H), 7.54 (d, 2H), 7.14 (d, 2H), 2.23 (s, 3H).

HPLC (Methode 1): Rₜ = 4.11 min.

MS (DCI, NH₃, *m*/*z*): 338 (M+H)⁺.

### Beispiel 19A

### 1-(4-Jodphenyl)-3-pyridin-4-yl-1H-pyrazol-4-carbaldehyd

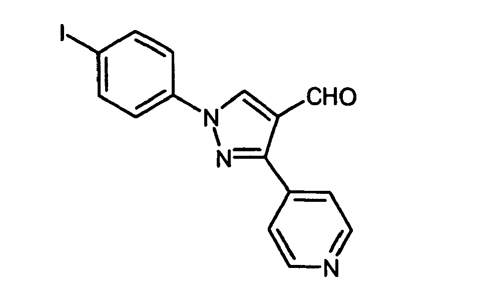

Analog zu dem in Beispiel 2A beschriebenen Verfahren werden aus 3.0 g (8.90 mmol) der Verbindung aus Beispiel 18A 468 mg (14% d. Th.) der Titelverbindung erhalten.

HPLC (Methode 6): Rₜ = 2.02 min.

MS (ESIpos, *m*/*z*): 376 (M+H)⁺.

### Beispiel 20A

### 1-(2,4-Dimethoxyphenyl)-N-{[1-(4-jodphenyl)-3-pyridin-4-yl-1H-pyrazol-4-yl]methyl}-methanamin

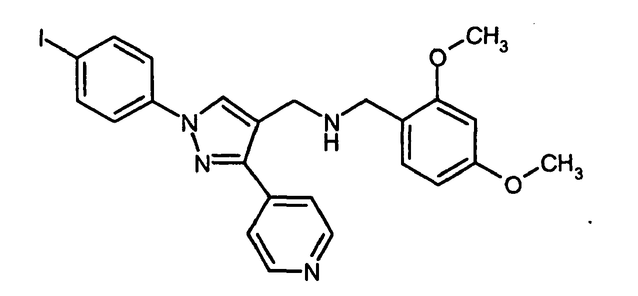

Analog zu dem unter Beispiel 3A beschriebenen Verfahren werden aus 468 mg (1.249 mmol) der Verbindung aus Beispiel 19A 657 mg (99% d. Th.) der Titelverbindung erhalten, die ohne weitere Aufreinigung in der nächsten Synthesestufe eingesetzt wird.

HPLC (Methode 3): Rₜ = 1.49 min (77%).

MS (ESIpos, *m*/*z*): 527 (M+H)⁺.

### Beispiel 21A

### 5-Chlor-N-(2,4-dimethoxybenzyl)-N-{[1-(4-jodphenyl)-3-pyridin-4-yl-1H-pyrazol-4-yl]methyl}-thiophen-2-carbonsäureamid

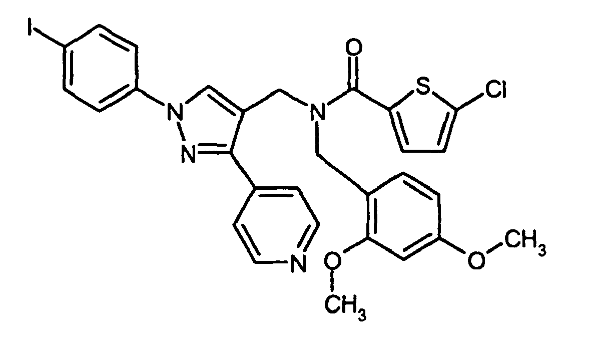

Analog zu dem unter Beispiel 4A beschriebenen Verfahren werden 657 mg (1.249 mmol) der Verbindung aus Beispiel 20A zu 405 mg (48% d. Th.) der Titelverbindung umgesetzt.

HPLC (Methode 5): Rₜ = 2.96 min (99%).

MS (ESIpos, *m*/*z*): 671/673 (³⁵Cl/³⁷Cl) (M+H)⁺.

### Beispiel 22A

### 5-Chlor-N-(2,4-dimethoxybenzyl)-N-({1-[4-(3-oxomorpholin-4-yl)phenyl]-3-pyridin-4-yl-1H-pyrazol-4-yl}methyl)thiophen-2-carbonsäureamid

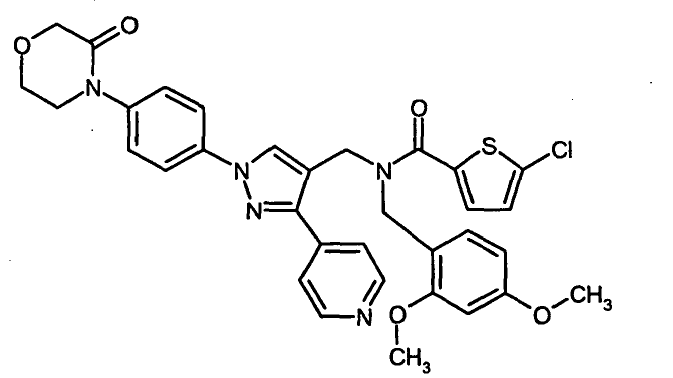

Analog zu dem unter Beispiel 5A beschriebenen Verfahren werden 150 mg (0.224 mmol) der Verbindung aus Beispiel 21 A zu 120 mg (84% d. Th.) der Titelverbindung umgesetzt.

¹H-NMR (400 MHz, DMSO-d₆, δ/*ppm*): 8.61 (d, 2H), 8.59 (breit, 1H), 7.98 (d, 2H), 7.64 (d, 2H), 7.57 (2H, d), 7.18 (breit, 1H), 7.09 (d, 1H), 7.03 (breit, 1H), 6.48 (breit, 1H), 6.45 (dd, 1H), 4.78 (breit, 2H), 4.62 (breit, 2H), 4.23 (s, 2H), 4.00 (t, 2H), 3.79 (t, 2H), 3.72 (s, 3H), 3.57 (s, breit, 3H).

HPLC (Methode 2): Rₜ = 4.20 min.

MS (DCI, NH₃, *m*/*z*): 644/646 (³⁵Cl/³⁷Cl) (M+H)⁺.

### Beispiel 23A

### 5-Chlor-N-(2,4-dimethoxybenzyl)-N-({1-[4-(2-oxopiperidin-1-yl)phenyl]-3-pyridin-4-yl-1H-pyrazol-4-yl}methyl)thiophen-2-carbonsäureamid

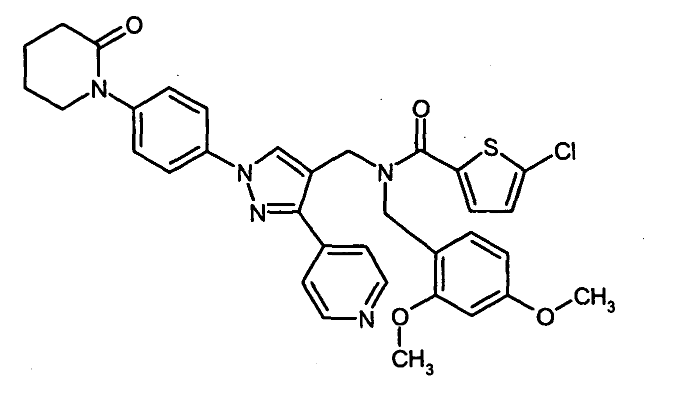

Analog zu dem unter Beispiel 5A beschriebenen Verfahren werden 248 mg (0.371 mmol) der Verbindung aus Beispiel 21A und Piperidin-2-on zu 66 mg (28% d. Th.) der Titelverbindung umgesetzt.

¹H-NMR (500 MHz, DMSO-d₆, δ/*ppm*): 8.61-8.53 (m, 2H), 8.13 (s, 1H), 7.93 (d, 2H), 7.62 (d, 2H), 7.43 (d, 2H), 7.18 (breit, 1H), 7.09 (d, 1H), 7.03 (breit, 1H), 6.47 (breit, 1H), 6.45 (dd, 1H), 4.78 (breit, 2H), 4.61 (breit, 2H), 3.71 (s, 3H), 3.65 (t, 2H), 3.57 (s, breit, 3H), 2.41 (t, 2H), 1.91-1.82 (m, 4H).

HPLC (Methode 2): Rₜ = 4.38 min.

MS (ESIpos, *m*/*z*): 642/644 (³⁵Cl/³⁷Cl) (M+H)⁺.

### Ausführungsbeispiele

### Beispiel 1

### 5-Chlor-N-({1-[4-(3-oxomorpholin-4-yl)phenyl]-3-phenyl-1H-pyrazol-4-yl}methyl)thiophen-2-carbonsäureamid

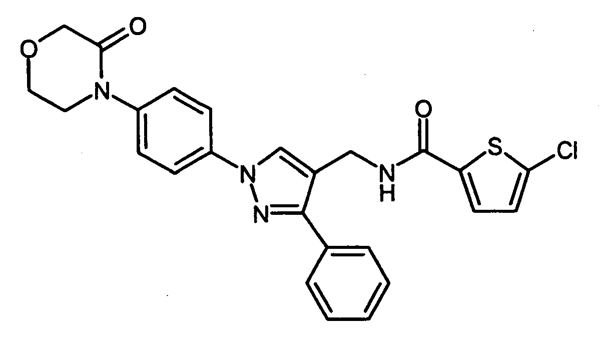

Eine Lösung von 39 mg (0.061 mmol) der Verbindung aus Beispiel 5A in 5 ml Dichlormethan wird mit 0.5 ml Trifluoressigsäure versetzt und 30 Minuten bei Raumtemperatur gerührt. Dann wird das Reaktionsgemisch zur Trockene eingedampft und der Rückstand in Acetonitril aufgenommen. Es wird vom Ungelösten abfiltriert und das Filtrat nach Einengen mittels präparativer HPLC (Methode 7) gereinigt. Es werden 26 mg (85% d. Th.) der Titelverbindung erhalten.

¹H-NMR (400 MHz, DMSO-d₆, δ/*ppm*): 9.05 (t, 1H), 8.54 (s, 1H), 7.93 (d, 2H), 7.71 (d, 2H), 7.70 (d, 1H), 7.55 (d, 2H), 7.48 (dd, 2H), 7.41 (dd, 1H), 7.19 (d, 1H), 4.53 (d, 2H), 4.23 (s, 2H), 4.00 (t, 2H), 3.78 (t, 2H).

HPLC (Methode 2): Rₜ = 4.57 min.

MS (ESIpos, *m*/*z*): 493/495 (³⁵Cl/³⁷Cl) (M+H)⁺.

### Beispiel 2

### 5-Chlor-N-({1-[3-methyl-4-(3-oxomorpholin-4-yl)phenyl]-3-phenyl-1H-pyrazol-4-yl}methyl)-thiophen-2-carbonsäureamid

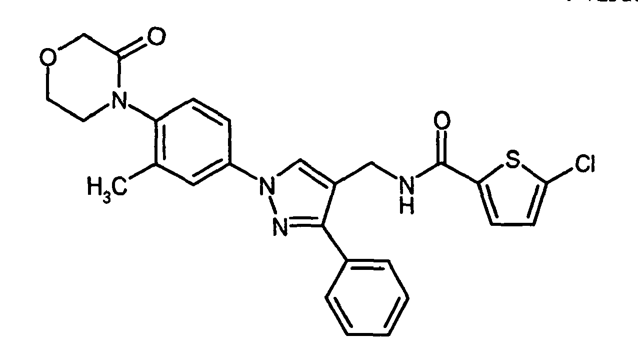

Analog zu dem unter Beispiel 1 beschriebenen Verfahren werden aus 88 mg (0.134 mmol) der Verbindung aus Beispiel 11A 58 mg (86% d. Th.) der Titelverbindung erhalten.

¹H-NMR (400 MHz, DMSO-d₆, δ/*ppm*): 9.01 (t, breit, 1H), 8.52 (s, 1H), 7.84 (d, 1H), 7.79-7.73 (m, 3H), 7.69 (d, 1H), 7.50-7.46 (m, 2H), 7.42-7.37 (m, 2H), 7.18 (d, 1H), 4.53 (d, 2H), 4.28-4.17 (m, 2H), 4.02-3.97 (m, 2H), 3.77-3.68 (m, 2H), 2.22 (s, 3H).

HPLC (Methode 2): Rₜ = 4.59 min.

MS (ESIpos, *m*/*z*): 507/509 (³⁵Cl/³⁷Cl) (M+H)⁺.

### Beispiel 3

### 5-Chlor-N-({1-[4-(3-oxomorpholin-4-yl)phenyl]-3-pyridin-3-yl-1H-pyrazol-4-yl}methyl)thiophen-2-carbonsäureamid-Hydrochlorid

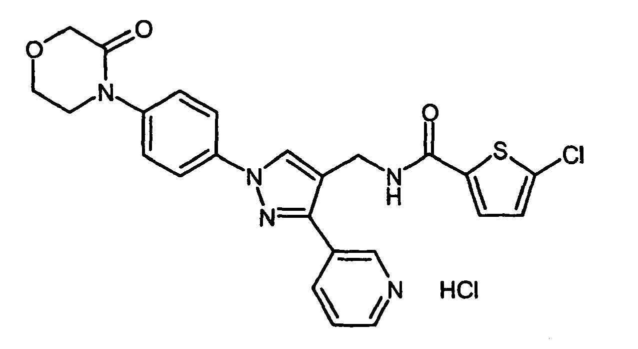

Analog zu dem unter Beispiel 1 beschriebenen Verfahren werden aus 115 mg (0.179 mmol) der Verbindung aus Beispiel 16A 71 mg (75% d. Th.) der Titelverbindung erhalten, die durch Lösen in Methanol und 1-molarer Salzsäure und anschließendes Eindampfen in das Hydrochloridsalz überführt wird.

¹H-NMR (400 MHz, DMSO-d₆, δ/*ppm*): 9.07-9.03 (m, 2H), 8.71 (d, 1H), 8.63 (s, 1H), 8.38 (d, 1H), 7.96 (d, 2H), 7.70 (dd, 1H), 7.67 (d, 1H), 7.58 (d, 2H), 7.17 (d, 1H), 4.58 (d, 2H), 4.23 (s, 2H), 4.00 (t, 2H), 3.78 (t, 2H).

HPLC (Methode 2): Rₜ = 3.76 min.

MS (ESIpos, *m*/*z*): 494/496 (³⁵Cl³⁷Cl) (M+H)⁺.

### Beispiel 4

### 5-Chlor-N-({1-[4-(2-oxopiperidin-1-yl)phenyl]-3-pyridin-3-yl-1H-pyrnol-4-yl}methyl)thiophen-2-carbonsäureamid-Hydrochlorid

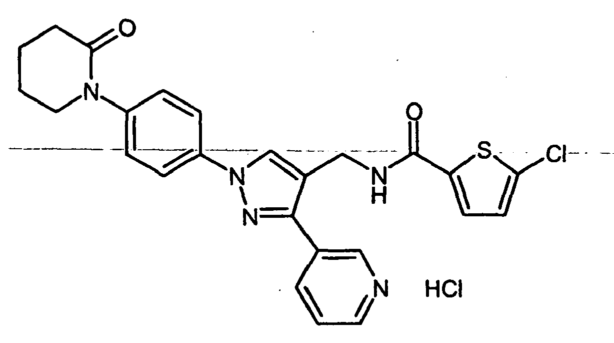

Analog zu dem unter Beispiel 1 beschriebenen Verfahren werden aus 68 mg (0.107 mmol) der Verbindung aus Beispiel 17A 44 mg (79% d. Th.) der Titelverbindung erhalten, die durch Lösen in Methanol und 1-molarer Salzsäure und anschließendes Eindampfen in das Hydrochloridsalz überführt wird.

¹H-NMR (500 MHz, DMSO-d₆, δ/*ppm*): 9.10 (s, 1H), 9.09 (t, 1H), 8.74 (dd, 1H), 8.63 (s, 1H), 8.48 (dd, 1H), 7.91 (d, 2H), 7.81-7.77 (m, 1H), 7.66 (d, 1H), 7.45 (d, 2H), 7.18 (d, 1H), 4.58 (d, 2H), 3.64 (t, 2H), 2.42 (t, 2H), 1.91-1.82 (m, 4H).

HPLC (Methode 2): Rₜ = 3.86 min.

MS (DCI, NH₃, *m*/*z*): 492/494 (³⁵Cl/³⁷Cl) (M+H)⁺.

### Beispiel 5

### 5-Chlor-N-({1-[4-(3-oxomorpholin-4-yl)phenyl]-3-pyridin-4-yl-1H-pyrazol-4-yl}methyl)thiophen-2-carbonsäureamid-Hydrochlorid

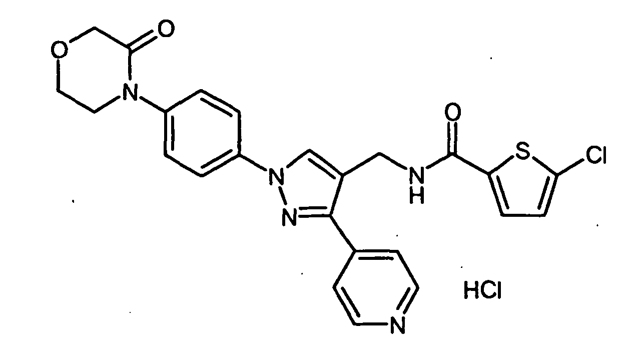

Analog zu dem unter Beispiel 1 beschriebenen Verfahren werden aus 108 mg (0.168 mmol) der Verbindung aus Beispiel 22A 66 mg (75% d. Th.) der Titelverbindung erhalten, die durch Lösen in Methanol und 1-molarer Salzsäure und anschließendes Eindampfen in das Hydrochloridsalz überführt wird.

¹H-NMR (500 MHz, DMSO-d₆, δ/*ppm*); 9.13 (1H), 8.85 (2H), 8.70 (1H), 8.18 (2H), 8.00 (2H), 7.68 (1H), 7.61 (2H), 7.19 (1H), 4.66 (2H), 4.23 (2H), 4.00 (2H), 3.80 (2H).

HPLC (Methode 2): Rₜ = 3.72 min.

MS (DCI, NH₃, *m*/*z*): 494/496 (³⁵Cl/³⁷Cl) (M+H)⁺.

### Beispiel 6

### 5-Chlor-N-({1-[4-(2-oxopiperidin-1-yl)phenyl]-3-pyridin-4-yl-1H-pyrazol-4-yl}methyl)thiophen-2-carbonsäureamid

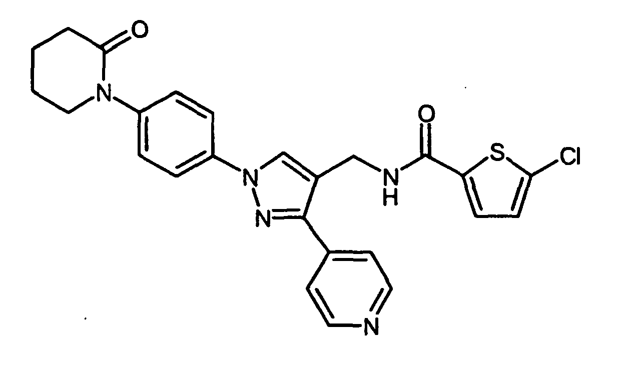

Analog zu dem unter Beispiel 1 beschriebenen Verfahren werden aus 65 mg (0.101 mmol) der Verbindung aus Beispiel 23A 36 mg (68% d. Th.) der Titelverbindung erhalten.

¹H-NMR (400 MHz, DMSO-d₆, δ/*ppm*): 9.10 (t, 1H), 8.81 (d, 2H), 8.64 (s, 1H), 8.10 (d, 2H), 7.94 (d, 2H), 7.67 (d, 1H), 7.47 (d, 2H), 7.18 (d, 1H), 4.64 (d, 2H), 3.65 (t, 2H), 2.42 (t, 2H), 1.93-1.82 (m, 4H).

HPLC (Methode 2): Rₜ = 3.82 min.

MS (ESIpos, *m*/*z*): 492/494 (³⁵Cl/³⁷Cl) (M+H)⁺.

### B. Bewertung der pharmakologischen Wirksamkeit

Die erfindungsgemäßen Verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung

Zur Bestimmung der Faktor Xa-Hemmung der oben aufgeführten Substanzen wird ein biochemisches Testsystem aufgebaut, in dem die Umsetzung eines Faktor Xa-Substrates zur Ermittlung der enzymatischen Aktivität von humanem Faktor Xa benutzt wird. Dabei spaltet Faktor Xa aus dem peptischen Substrat Aminomethylcoumarin ab, das fluoreszent gemessen wird. Die Bestimmungen werden in Mikrotiterplatten durchgeführt.

Zu testende Substanzen werden in unterschiedlichen Konzentrationen in Dimethylsulfoxid gelöst und 15 min mit humanem Faktor Xa (1.3 nmol/l gelöst in 50 mmol/l Tris-Puffer [C,C,C-Tris(hydroxymethyl)-aminomethan], 100 mmol/L NaCl, 0.1% BSA [bovines Serumalbumin], pH 7.4) bei 22°C inkubiert. Anschließend wird das Substrat (5 µmol/l Boc-Ile-Glu-Gly-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

Repräsentative Wirkdaten aus diesem Test sind in der folgenden Tabelle 1 aufgeführt:

**Tabelle 1**

| **Beispiel Nr.** | **IC₅₀ [nM]** |
|---|---|
| 1 | 0.7 |
| 2 | 0.7 |
| 3 | 0.8 |

### a.2) Bestimmung der Selektivität

Zum Nachweis der Selektivität der Substanzen bezüglich Faktor Xa -Hemmung werden die Prüfsubstanzen auf ihre Hemmung anderer humaner Serinproteasen wie Trypsin und Plasmin hin untersucht. Zur Bestimmung der enzymatischen Aktivität von Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l NaCl, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Trypsin, 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethylenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über eine Ohrvene oder oral mittels Schlundsonde verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel 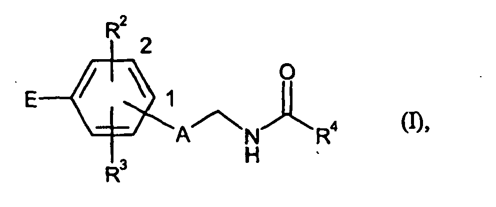 in welcher
E für eine Gruppe der Formel 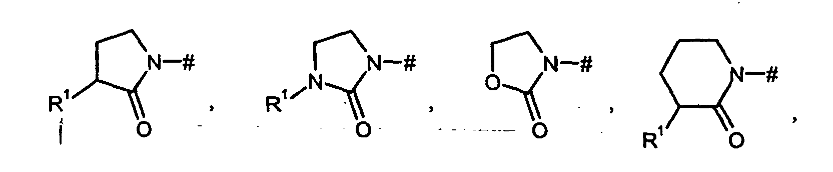 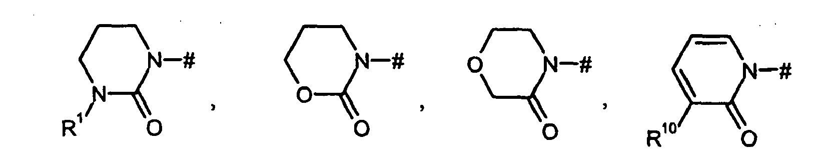 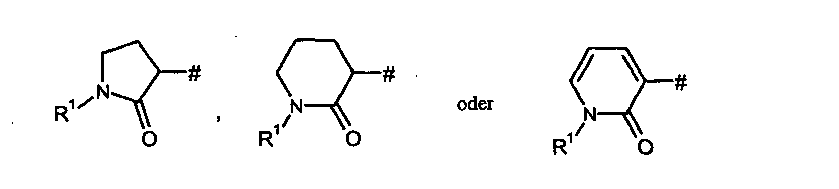 steht,
wobei
# die Anknüpfstelle an den Phenylring ist,
R¹ für Wasserstoff, Hydroxy, Amino, C₁-C₄-Alkyl, C₁-C₄-Alkoxy, C₁-C₄-Alkylamino, C₃-C₆-Cycloalkyl, C₃-C₆-Cycloalkyloxy, C₃-C₆-Cycloalkylamino, C₁-C₄-Alkylcarbonylamino oder C₁-C₄-Alkoxycarbonylamino steht,
worin
Alkyl, Alkoxy, Alkylamino substituiert sein können mit einem Substituenten, wobei der Substituent ausgewählt wird aus der Gruppe bestehend aus Hydroxy, Amino, C₁-C₄-Alkoxy, C₁-C₄-Alkylamino, C₃-C₆-Cycloalkylamino und einem 4- bis 7-gliedrigen gesättigten, über ein N-Atom gebundenen Heterocyclyl, das ein Ringglied aus der Reihe N-R⁵ oder O enthalten kann,
worin
R⁵ für Wasserstoff oder C₁-C₄-Alkyl steht,
und
R¹⁰ für Wasserstoff, C₁-C₄-Alkyl, C₁-C₄-Alkoxy, C₃-C₆-Cycloallcyl, C₃-C₆-Cycloalkyloxy oder -NR¹¹R¹² steht,
wobei
R¹¹ für C₁-C₄-Alkyl oder C₃-C₆-Cycloalkyl steht,
und
R¹² für C₁-C₄-Alkyl steht,
A für ein 5-gliedriges Heteroaryl oder teilweise ungesättigtes 5-gliedriges Heterocyclyl steht,
wobei Heteroaryl und Heterocyclyl in 1 oder 2 Position an den Phenyl-Ring gebunden sind und Heteroaryl und Heterocyclyl selber eine 1,3-Verknüpfung mit dem Phenyl-Ring und der Carbonylaminomethyl-Gruppe aufweisen,
und
wobei Heteroaryl und Heterocyclyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl oder ein 5- oder 6-gliedriges Heteroaryl steht,
wobei Phenyl und Heteroaryl substituiert sein können mit 1 bis 3 Substituenten, wobei die Substituenten unabhängig voneinander ausgewählt werden aus der Gruppe bestehend aus Halogen, Hydroxy, Hydroxymethyl, Hydroxyethyl, Amino, Aminomethyl, Aminoethyl, C₁-C₄-Alkyl, C₁-C₄-Alkoxy, C₁-C₄-Alkoxymethyl, C₁-C₄-Alkylamino, C₁-C₄-Alkylanünomethyl, Hydroxycarbonyl, Hydroxycarbonylmethyl, Aminocarbonyl, Aminocarbonylmethyl, C₁-C₄-Alkoxycarbonyl, C₁-C₄-Alkoxycarbonylmethyl, C₁-C₄-Alkylaminocarbonyl, C₁-C₄-Alkylaminocarbonylmethyl, Aminosulfonyl, C₁-C₄-Alkylaminosulfonyl und C₁-C₄-Alkylsulfonyl,
R² für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkoxymethyl, C₁-C₄-Alkylarnino, C₃-C₆-Cycloallcyl, Aminocarbonyl, C₁-C₄-Alkoxycarbonyl oder C₁-C₄-Allcylaminocarbonyl steht,
R³ für Wasserstoff, Fluor, Chlor, Cyano, Hydroxy, Amino, Trifluormethyl, Trifluormethoxy, C₁-C₄-Mkyl, C₁-C₄-Allcoxy, C₁-C₄-Alkoxymethyl, C₁-C₄-Alkylamino, C₃-C₆-Cycloallcyl, Aminocarbonyl, C₁-C₄-Alkoxycarbonyl oder C₁-C₄-AlKylaminocarbonyl steht,
R⁴ für eine Gruppe der Formel 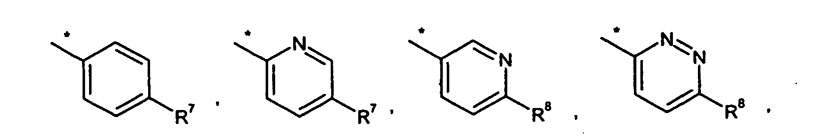 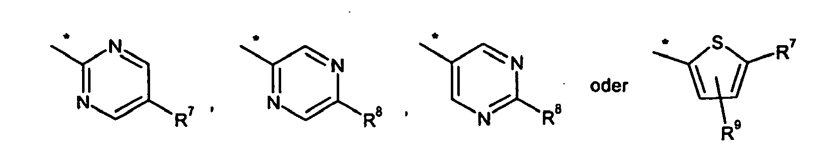 steht,
wobei
* die Anknüpfstelle an die Carbonylgruppe ist,
R⁷ für Wasserstoff, Fluor, Chlor, Cyano, Ethinyl, C₁-C₄-Alkyl, C₁-C₄-Alkoxy oder C₃-C₆-Cycloalkyl steht,
R⁸ für Wasserstoff, Amino, C₁-C₄-Alkyl, C₁-C₄-Alkylamino oder C₃-C₆-Cycloalkyl steht,
und
R⁹ für Wasserstoff, Fluor, Chlor, Amino oder C₁-C₄-Alkyl steht,
oder eines ihrer Salze, ihrer Solvate oder der Solvate ihrer Salze.

2. Verbindung nach Anspruch 1, **dadurch gekennzeichnet, dass**
E für eine Gruppe der Formel 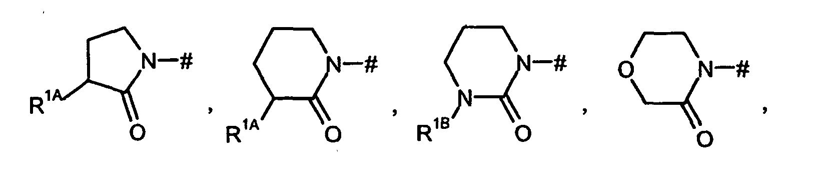 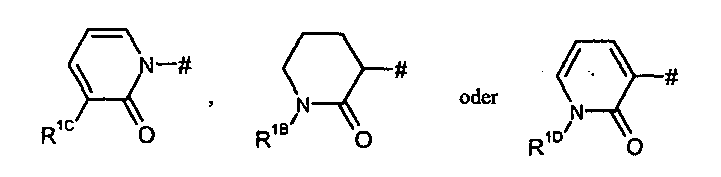 steht,
wobei
# die Anknüpfstelle an den Phenylring ist,
R^{1A} für Wasserstoff, Hydroxy, Hydroxymethyl, 2-Hydroxyethyl, Amino oder Methoxy steht,
R^{1B} für Wasserstoff, Hydroxy, Amino, Methyl oder Ethyl steht,
worin
Methyl substituiert sein kann mit einem Substituenten Pyrrolidin-1-yl,
und
Ethyl substituiert sein kann mit einem Substituenten, wobei der Substituent ausgewählt wird aus der Gruppe bestehend aus Hydroxy, Amino, Dimethylamino und Cyclopropylamino,
R^{1C} für Wasserstoff, Methyl oder Ethyl steht,
worin
Methyl substituiert sein kann mit einem Substituenten, wobei der Substituent ausgewählt wird aus der Gruppe bestehend aus Hydroxy und Pyrrolidin-1-yl,
und
Ethyl substituiert sein kann mit einem Substituenten, wobei der Substituent ausgewählt wird aus der Gruppe bestehend aus Hydroxy, Amino und Cyclopropylamino,
und
R^{1D} für Wasserstoff, Methyl oder Ethyl steht,
worin
Methyl substituiert sein kann mit einem Substituenten, wobei der Substituent ausgewählt wird aus der Gruppe bestehend aus Cyclopropylamino und Pyrrolidin-1-yl,
und
Ethyl substituiert sein kann mit einem Substituenten, wobei der Substituent ausgewählt wird aus der Gruppe bestehend aus Hydroxy, Amino und Cyclopropylamino,
A für ein 5-gliedriges Heteroaryl oder teilweise ungesättigtes 5-gliedriges Heterocyclyl steht,
wobei Heteroaryl und Heterocyclyl in 1 oder 2 Position an den Phenyl-Ring gebunden sind und Heteroaryl und Heterocyclyl selber eine 1,3-Verknüpfung mit dem Phenyl-Ring und der. Carbonylaminomethyl-Gruppe aufweisen,
und
wobei Heteroaryl und Heterocyclyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl oder ein 5- oder 6-gliedriges Heteroaryl steht,
wobei Phenyl und Heteroaryl substituiert sein können mit 1 bis 3 Substituenten, wobei die Substituenten unabhängig voneinander ausgewählt werden aus der Gruppe bestehend aus Halogen, Hydroxy, Amino, C₁-C₄-Alkyl, C₁-C₄-Alkoxy, C₁-C₄-Alkoxymethyl, C₁-C₄-Alkylamino, C₁-C₄-Alkylaminomethyl, Hydroxycarbonyl, Hydroxycarbonylmethyl, Aminocarbonyl, Aminocarbonylmethyl, C₁-C₄-Alkoxycarbonyl, C₁-C₄-Alkoxycarbonylmethyl, C₁-C₄-Alkylaminocarbonyl, C₁-C₄-Alkylaminocarbonylmethyl, Aminosulfonyl, C₁-C₄-Alkylaminosulfonyl und C₁-C₄-Alkylsulfonyl,
R² für Wasserstoff, Fluor, Chlor, Cyano, Hydroxy, C₁-C₄-Alkyl oder C₁-C₄-Alkoxy steht,
R³ für Wasserstoff, Fluor, Chlor, Cyano, Hydroxy, C₁-C₄-Alkyl, C₁-C₄-Allcoxy, C₁-C₄-Alkoxymethyl, Cyclopropyl, Aminocarbonyl, C₁-C₄-Alkoxycarbonyl oder C₁-C₄-Alkylaminocarbonyl steht,
R⁴ für eine Gruppe der Formel 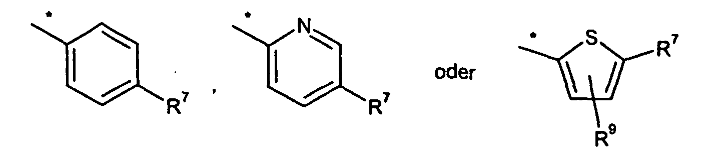 steht,
wobei
* die Anknüpfstelle an die Carbonylgruppe ist,
R⁷ für Fluor, Chlor, Ethinyl, Methyl oder Methoxy steht,
und
R⁹ für Wasserstoff steht.

3. Verbindung nach einem der Ansprüche 1 oder 2, **dadurch gekennzeichnet, dass**
E für eine Gruppe der Formel 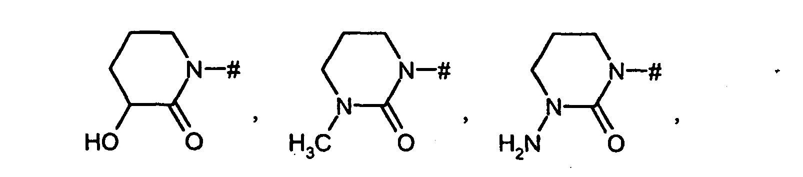 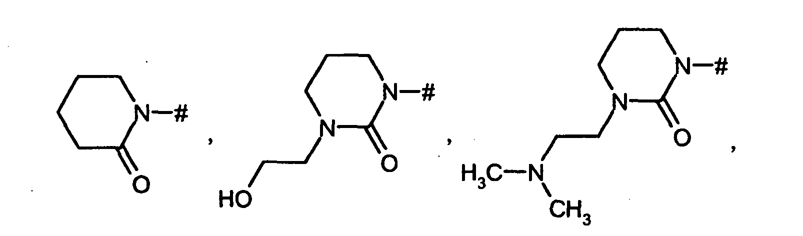 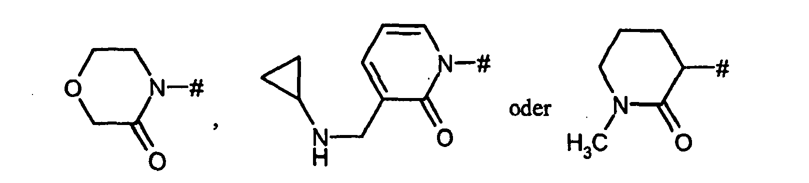 steht,
wobei
# die Anknüpfstelle an den Phenylring ist,
A für Pyrazolyl, Oxadiazolyl oder Isoxazolinyl steht,
wobei Pyrazolyl, Oxadiazolyl und Isoxazolinyl in 1 Position an den Phenyl-Ring gebunden sind und Pyrazolyl, Oxadiazolyl und Isoxazolinyl selber eine 1,3-Verknüpfung mit dem Phenyl-Ring und der Carbonylaminomethyl-Gruppe aufweisen,
und
wobei Pyrazolyl, Oxadiazolyl und Isoxazolinyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl, Pyrid-2-yl, Pyrid-3-yl, Pyrid-4-yl, 1,3-Oxazol-2-yl oder
Pyrimidin-2-yl steht,
wobei Phenyl, Pyrid-2-yl, Pyrid-3-yl, Pyrid-4-yl, 1,3-Oxazol-2-yl und Pyrimidin-2-yl substituiert sein können mit 1 bis 3 Substituenten, wobei die Substituenten unabhängig voneinander ausgewählt werden aus der Gruppe bestehend aus Halogen, Hydroxy, Amino, C₁-C₄-Alkyl, C₁-C₄-Alkoxy, C₁-C₄-Alkylamino, Hydroxycarbonyl, Aminocarbonyl, C₁-C₄-Alkoxycarbonyl, C₁-C₄-Alkylaminocarbonyl, Aminosulfonyl, C₁-C₄-Alkylaminosulfonyl und C₁-C₄-Alkylsulfonyl,
R² für Wasserstoff oder Fluor steht,
R³ für Wasserstoff, Fluor, Chlor, Cyano, Methyl, Ethyl, n-Propyl, iso-Propyl, Methoxy, Ethoxy, Methoxymethyl oder Cyclopropyl steht,
R⁴ für eine Gruppe der Formel 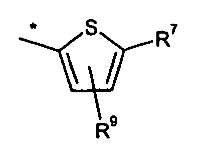 steht,
wobei
* die Anknüpfstelle an die Carbonylgruppe ist,
R⁷ für Fluor, Chlor oder Methyl steht,
und
R⁹ für Wasserstoff steht.

4. Verbindung nach einem der Ansprüche 1 bis 3, **dadurch gekennzeichnet, dass**
E für eine Gruppe der Formel 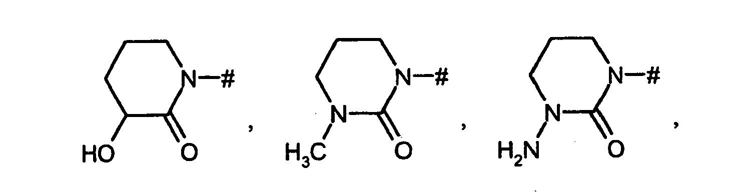 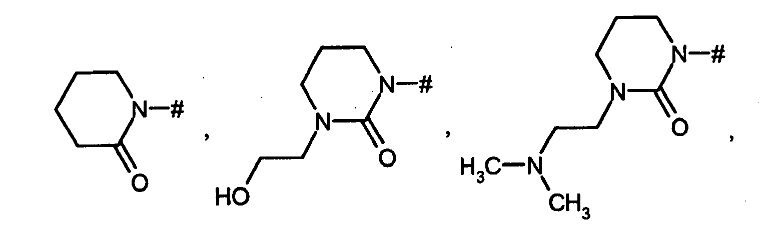 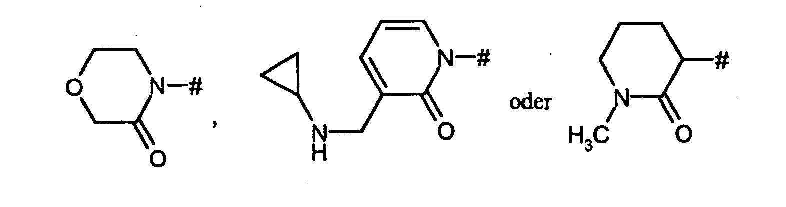 steht,
wobei
# die Anknüpfstelle an den Phenylring ist,
A für Pyrazolyl, Oxadiazolyl oder Isoxazolinyl steht,
wobei Pyrazolyl, Oxadiazolyl und Isoxazolinyl in 1 Position an den Phenyl-Ring gebunden sind und Pyrazolyl, Oxadiazolyl und Isoxazolinyl selber eine 1,3-Verknüpfung mit dem Phenyl-Ring und der Carbonylaminomethyl-Gruppe aufweisen,
und
wobei Pyrazolyl, Oxadiazolyl und Isoxazolinyl substituiert sind mit einem Substituenten R⁶,
wobei R⁶ am Nachbaratom des Atoms gebunden ist, an das die Carbonylaminomethyl-Gruppe gebunden ist, und eine 1,4-Verknüpfung zum Phenyl-Ring aufweist
und
wobei das Atom, an das R⁶ gebunden ist, ein Stickstoff- oder Kohlenstoffatom ist
und
wobei
R⁶ für Phenyl, Pyrid-2-yl, Pyrid-3-yl oder Pyrid-4-yl steht,
wobei Phenyl, Pyrid-2-yl, Pyrid-3-yl und Pyrid-4-yl substituiert sein können mit einem Substituenten, wobei der Substituent ausgewählt wird aus der Gruppe bestehend aus Halogen, Hydroxy, Amino, C₁-C₄-Alkyl, C₁-C₄-Alkoxy, C₁-C₄-Alkylamino, Hydroxycarbonyl, Aminocarbonyl, C₁-C₄-Alkoxycarbonyl und C₁-C₄-Alkylaminocarbonyl,
R² für Wasserstoff oder Fluor steht,
R³ für Wasserstoff, Fluor, Chlor, Methyl oder Methoxy steht,
R⁴ für eine Gruppe der Formel 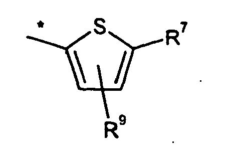 steht,
wobei
* die Anknüpfstelle an die Carbonylgruppe ist,
R⁷ für Chlor steht,
und
R⁹ für Wasserstoff steht.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass** eine Verbindung der Formel 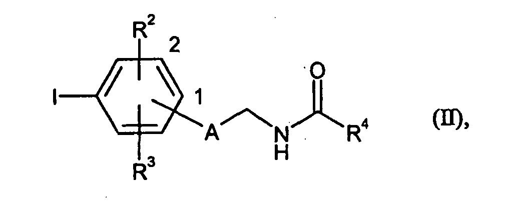 in welcher A, R², R³ und R⁴ die in Anspruch 1 angegebene Bedeutung haben,
mit einer Verbindung der Formel
E-H (III),
in welcher E die in Anspruch 1 angegebene Bedeutung hat,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von thromboembolischen Erkrankungen.

9. Arzneimittel enthaltend eine Verbindung nach einem der Ansprüche 1 bis 4 in Kombination mit einem inerten, nichttoxischen, pharmazeutisch geeigneten Hilfsstoff.

10. Arzneimittel enthaltend eine Verbindung nach einem der Ansprüche 1 bis 4 in Kombination mit einem weiteren Wirkstoff.

11. Arzneimittel nach Anspruch 9 oder 10 zur Behandlung und/oder Prophylaxe von thromboembolischen Erkrankungen.

## Claims

1. Compound of the formula 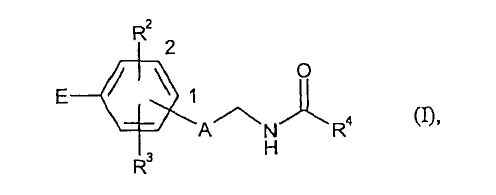 in which
E represents a group of the formula 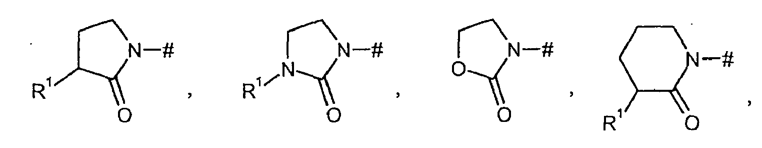 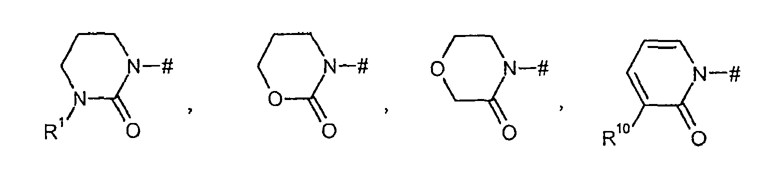 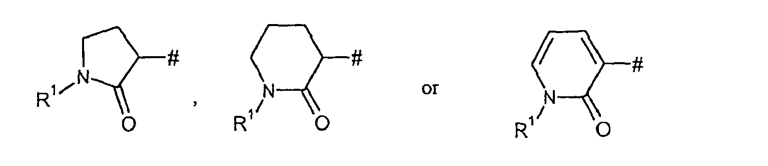 where
# is the point of attachment to the phenyl ring,
R¹ represents hydrogen, hydroxy, amino, C₁-C₄-alkyl, C₁-C₄-alkoxy, C₁-C₄-alkylamino, C₃-C₆-cycloalkyl, C₃-C₆-cycloalkyloxy, C₃-C₆-cycloalkylamino, C₁-C₄-alkylcarbonylamino or C₁-C₄-alkoxycarbonylamino,
where
alkyl, alkoxy, alkylamino may be substituted by a substituent, the substituent being selected from the group consisting of hydroxy, amino, C₁-C₄-alkoxy, C₁-C₄-alkylamino, C₃-C₆-cycloalkylamino and a 4- to 7-membered saturated heterocyclyl bound via an N atom which may contain a ring member from the group consisting of N-R⁵ or O,
where
R⁵ represents hydrogen or C₁-C₄-alkyl,
and
R¹² represents C₁-C₄-alkyl,
R¹⁰ represents hydrogen, C₁-C₄-alkyl, C₁-C₄-alkoxy, C₃-C₆-cycloalkyl, C₃-C₆-cycloalkyloxy or -NR¹¹R¹²,
where
R¹¹ represents C₁-C₄-alkyl or C₃-C₆-cycloalkyl,
and
R¹² represents C₁-C₄-alkyl,
A represents a 5-membered heteroaryl or partially unsaturated 5-membered heterocyclyl,
where heteroaryl and heterocyclyl are attached in the 1- or 2-position to the phenyl ring and heteroaryl and heterocyclyl for their part have a 1,3-attachment to the phenyl ring and the carbonylaminomethyl group,
and
where heteroaryl and heterocyclyl are substituted by a substituent R⁶,
where R⁶ is attached to the neighbouring atom of the atom to which the carbonylaminomethyl group is attached and has a 1,4-attachment to the phenyl ring
and
where the atom to which R⁶ is attached is a nitrogen or carbon atom
and
where
R⁶ represents phenyl or a 5- or 6-membered heteroaryl,
where phenyl and heteroaryl may be substituted by 1 to 3 substituents, where the substituents independently of one another are selected from the group consisting of halogen, hydroxy, hydroxymethyl, hydroxyethyl, amino, aminomethyl, aminoethyl, C₁-C₄-alkyl, C₁-C₄-alkoxy, C₁-C₄-alkoxymethyl, C₁-C₄-alkylamino, C₁-C₄-alkylaminomethyl, hydroxycarbonyl, hydroxycarbonylmethyl, aminocarbonyl, aminocarbonylmethyl, C₁-C₄-alkoxycarbonyl, C₁-C₄-alkoxycarbonylmethyl, C₁-C₄-alkylaminocarbonyl, C₁-C₄-alkylaminocarbonylmethyl, aminosulphonyl, C₁-C₄-alkylaminosulphonyl and C₁-C₄-alkylsulphonyl,
R² represents hydrogen, fluorine, chlorine, cyano, hydroxy, amino, trifluoromethyl, trifluoromethoxy, C₁-C₄-alkyl, C₁-C₄-alkoxy, C₁-C₄-alkoxymetbyl, C₁-C₄-alkylamino, C₃-C₆-cycloalkyl, aminocarbonyl, C₁-C₄-alkoxycarbonyl or C₁-C₄-alkylaminocarbonyl,
R³ represents hydrogen, fluorine, chlorine, cyano, hydroxy, amino, trifluoromethyl, trifluoromethoxy, C₁-C₄-alkyl, C₁-C₄-alkoxy, C₁-C₄-alkoxymethyl, C₁-C₄-alkylamino, C₃-C₆-cycloalkyl, aminocarbonyl, C₁-C₄-alkoxycarbonyl or C₁-C₄-alkylaminocarbonyl,
R⁴ represents a group of the formula 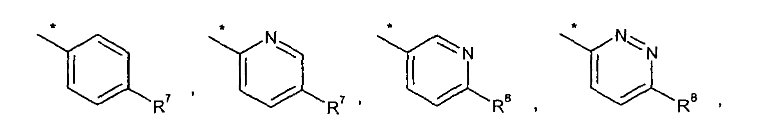 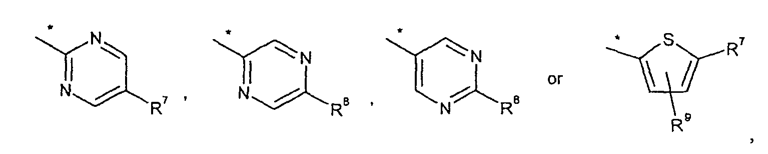 where
* xis the point of attachment to the carbonyl group,
R⁷ represents hydrogen, fluorine, chlorine, cyano, ethynyl, C₁-C₄-alkyl, C₁-C₄-alkoxy or C₃-C₆-cycloalkyl,
R⁸ represents hydrogen, amino, C₁-C₄-alkyl, C₁-C₄-alkylamino or C₃-C₆-cycloalkyl,
and
R⁹ represents hydrogen, fluorine, chlorine, amino or C₁-C₄-alkyl,
or one of its salts, its solvates or the solvates of its salts.

2. Compound according to Claim 1, **characterized in that**
E represents a group of the formula 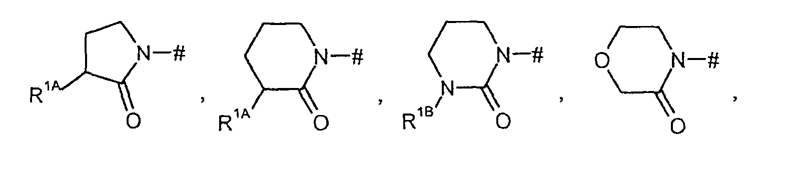 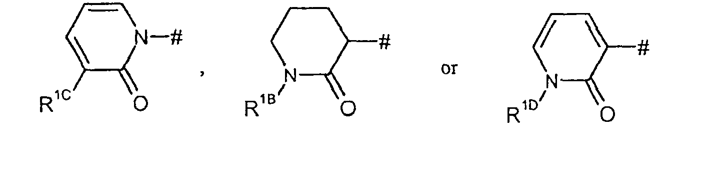 where
# is the point of attachment to the phenyl ring,
R^{1A} represents hydrogen, hydroxy, hydroxymethyl, 2-hydroxyethyl, amino or methoxy,
R^{1B} represents hydrogen, hydroxy, amino, methyl or ethyl,
where
methyl may be substituted by a pyrrolidin-1-yl substituent,
and
ethyl may be substituted by a substituent, where the substituent is selected from the group consisting of hydroxy, amino, dimethylamino and cyclopropylamino,
R^{1C} represents hydrogen, methyl or ethyl,
in which
methyl may be substituted by a substituent, where the substituent is selected from the group consisting of hydroxy and pyrrolidin-1-yl,
and
ethyl may be substituted by a substituent, where the substituent is selected from the group consisting of hydroxy, amino and cyclopropylamino,
and
R^{1D} represents hydrogen, methyl or ethyl,
in which
methyl may be substituted by a substituent, where the substituent is selected from the group consisting of cyclopropylamino and pyrrolidin-1-yl,
and
ethyl may be substituted by a substituent, where the substituent is selected from the group consisting of hydroxy, amino and cyclopropylamino,
A represents a 5-membered heteroaryl or partially unsaturated 5-membered heterocyclyl,
where heteroaryl and heterocyclyl are attached in the 1- or 2-position to the phenyl ring and heteroaryl and heterocyclyl for their part have a 1,3-attachment to the phenyl ring and the carbonylaminomethyl group,
and
where heteroaryl and heterocyclyl are substituted by a substituent R⁶,
where R⁶ is attached to the neighbouring atom of the atom to which the carbonylaminomethyl group is attached and has a 1,4-attachment to the phenyl ring
and
where the atom to which R⁶ is attached is a nitrogen or carbon atom
and
where
R⁶ represents phenyl or a 5- or 6-membered heteroaryl,
where phenyl and heteroaryl may be substituted by 1 to 3 substituents, where the substituents independently of one another are selected from the group consisting of halogen, hydroxy, amino, C₁-C₄-alkyl, C₁-C₄-alkoxy, C₁-C₄-alkoxymethyl, C₁-C₄-alkylamino, C₁-C₄-alkylaminomethyl, hydroxycarbonyl, hydroxycarbonylmethyl, aminocarbonyl, aminocarbonylmethyl, C₁-C₄-alkoxycarbonyl, C₁-C₄-alkoxycarbonylmethyl, C₁-C₄-alkylaminocarbonyl, C₁-C₄-alkylaminocarbonylmethyl, aminosulphonyl, C₁-C₄-alkylaminosulphonyl and C₁-C₄-alkylsulphonyl,
R² represents hydrogen, fluorine, chlorine, cyano, hydroxy, C₁-C₄-alkyl or C₁-C₄-alkoxy,
R³ represents hydrogen, fluorine, chlorine, cyano, hydroxy, C₁-C₄-alkyl, C₁-C₄-alkoxy, C₁-C₄-alkoxyrnethyl, cyclopropyl, aminocarbonyl, C₁-C₄-alkoxycarbonyl or C₁-C₄-alkylaminocarbonyl,
R⁴ represents a group of the formula 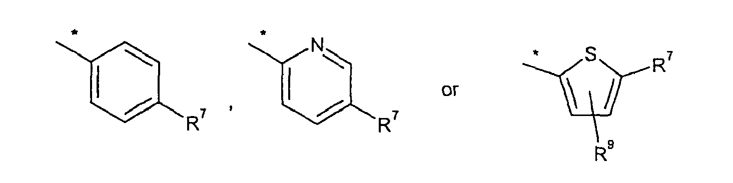 where
* is the point of attachment to the carbonyl group,
R⁷ represents fluorine, chlorine, ethynyl, methyl or methoxy,
and
R⁹ represents hydrogen.

3. Compound according to Claim 1 or 2, **characterized in that**
E represents a group of the formula 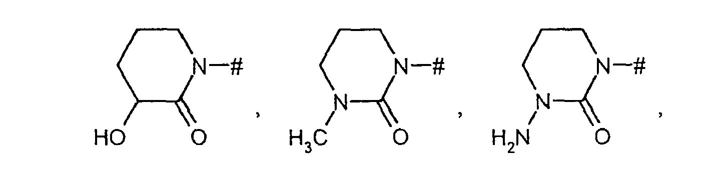 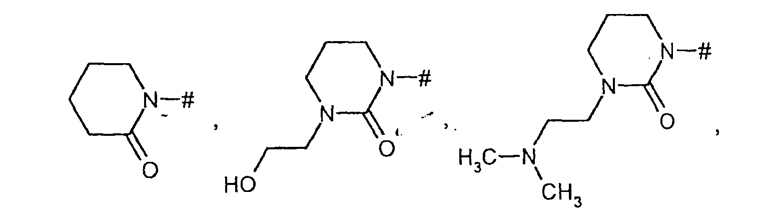 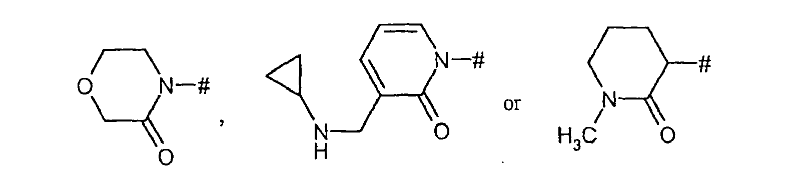 where
# is the point of attachment to the phenyl ring,
A represents pyrazolyl, oxadiazolyl or isoxazolinyl,
where pyrazolyl, oxadiazolyl and isoxazolinyl are attached in the 1-position to the phenyl ring and pyrazolyl, oxadiazolyl and isoxazolinyl for their part have a 1,3-attachment to the phenyl ring and the carbonylaminomethyl group,
and
where pyrazolyl, oxadiazolyl and isoxazolinyl are substituted by a substituent R⁶,
where R⁶ is attached to the neighbouring atom of the atom to which the carbonylaminomethyl group is attached and has a 1,4-attachment to the phenyl ring
and
where the atom to which R⁶ is attached is a nitrogen or carbon atom
and
where
R⁶ represents phenyl, pyrid-2-yl, pyrid-3-yl, pyrid-4-yl, 1,3-oxazol-2-yl or pyrimidin-2-yl,
where phenyl, pyrid-2-yl, pyrid-3-yl, pyrid-4-yl, 1,3-oxazol-2-yl and pyrimidin-2-yl may be substituted by 1 to 3 substituents, where the substituents independently of one another are selected from the group consisting of halogen, hydroxy, amino, C₁-C₄-alkyl, C₁-C₄-alkoxy, C₁-C₄-alkylamino, hydroxycarbonyl, aminocarbonyl, C₁-C₄-alkoxycarbonyl, C₁-C₄-alkylaminocarbonyl, aminosulphonyl, C₁-C₄-alkylaminosulphonyl and C₁-C₄-alkylsulphonyl,
R² represents hydrogen or fluorine,
R³ represents hydrogen, fluorine, chlorine, cyano, methyl, ethyl, n-propyl, isopropyl, methoxy, ethoxy, methoxymethyl or cyclopropyl,
R⁴ represents a group of the formula 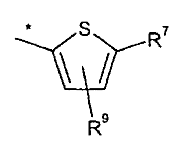 where
* is the point of attachment to the carbonyl group,
R⁷ represents fluorine, chlorine or methyl,
and
R⁹ represents hydrogen.

4. Compound according to any of Claims 1 to 3, **characterized in that**
E represents a group of the formula 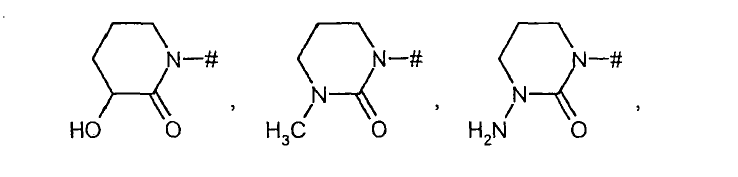 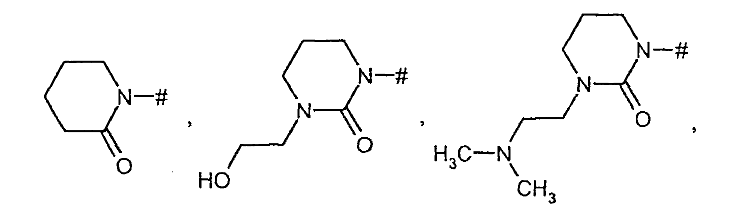 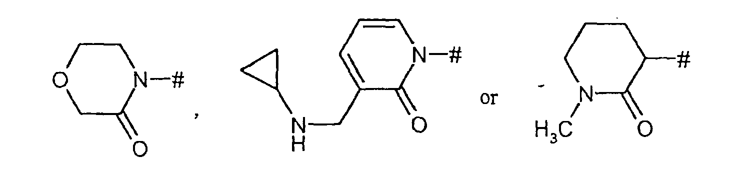 where
# is the point of attachment to the phenyl ring,
A represents pyrazolyl, oxadiazolyl or isoxazolinyl,
where pyrazolyl, oxadiazolyl and isoxazolinyl are attached in the 1-position to the phenyl ring and pyrazolyl, oxadiazolyl and isoxazolinyl for their part have a 1,3-attachment to the phenyl ring and the carbonylaminomethyl group,
and
where pyrazolyl, oxadiazolyl and isoxazolinyl are substituted by a substituent R⁶,
where R⁶ is attached to the neighbouring atom of the atom to which the carbonylaminomethyl group is attached and has a 1,4-attachment to the phenyl ring
and
where the atom to which R⁶ is attached is a nitrogen or carbon atom
and
where
R⁶ represents phenyl, pyrid-2-yl, pyrid-3-yl or pyrid-4-yl,
-where phenyl, pyrid-2-yl, pyrid-3-yl and pyrid-4-yl may be substituted by a substituent, where the substituent is selected from the group consisting of halogen, hydroxy, amino, C₁-C₄-alkyl, C₁-C₄-alkoxy, C₁-C₄-alkylamino, hydroxycarbonyl, aminocarbonyl, C₁-C₄-alkoxycarbonyl and C₁-C₄-alkylaminocarbonyl,
R² represents hydrogen or fluorine,
R³ represents hydrogen, fluorine, chlorine, methyl or methoxy,
R⁴ represents a group of the formula 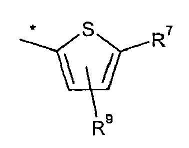 where
* is the point of attachment to the carbonyl group,
R⁷ represents chlorine,
and
R⁹ represents hydrogen.

5. Process for preparing a compound of the formula (I) or one of its salts, its solvates or the solvates of its salts according to Claim 1, **characterized in that** a compound of the formula 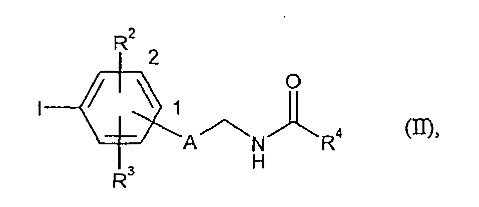 in which A, R², R³ and R⁴ have the meaning given in Claim 1,
is reacted with a compound of the formula
E-H (III),
in which E has the meaning given in Claim 1.

6. Compound according to any of Claims 1 to 4 for the treatment and/or prophylaxis of diseases.

7. Use of a compound according to any of Claims 1 to 4 for preparing a medicament for the treatment and/or prophylaxis of diseases.

8. Use of a compound according to any of Claims 1 to 4 for preparing a medicament for the treatment and/or prophylaxis of thromboembolic disorders.

9. Medicament, comprising a compound according to any of Claims 1 to 4 in combination with an inert non-toxic pharmaceutically acceptable auxiliary.

10. Medicament comprising a compound according to any of Claims 1 to 4 in combination with a further active compound.

11. Medicament according to Claim 9 or 10 for the treatment and/or prophylaxis of thromboembolic disorders.

## Revendications

1. Composé de la formule 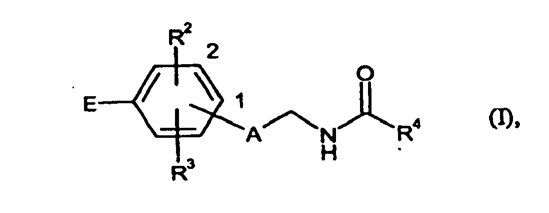 dans laquelle
E représente un groupe de la formule 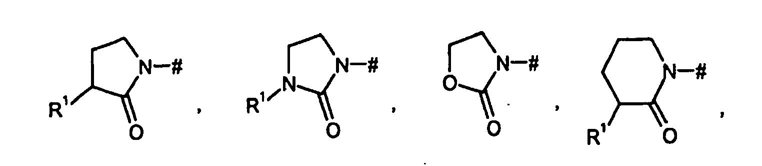 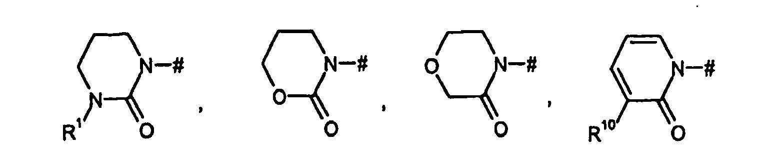 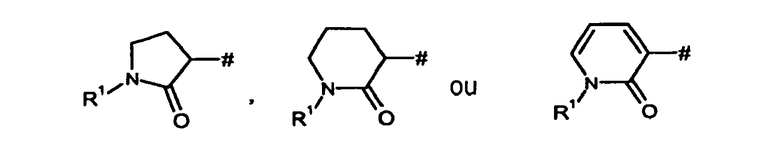 où
# est la position de liaison sur le noyau phényle,
R¹ représente l'hydrogène, un groupe hydroxy, amino, alkyle en C₁-C₄, alcoxy en C₁-C₄, alkylamino en C₁-C₄, cycloalkyle en C₃-C₆, cycloalkyloxy en C₃-C₆, cycloalkylamino en C₃-C₆, alkylcarbonylamino en C₁-C₄ ou alcoxycarbonylamino en C₁-C₄,
dans lequel
le groupe alkyle, alcoxy, alkylamino peut être substitué avec un substituant, le substituant étant choisi dans le groupe constitué d'un groupe hydroxy, amino, alcoxy en C₁-C₄, alkylamino en C₁-C₄, cycloalkylamino en C₃-C₆ et un groupe hétérocyclyle saturé à de 4 à 7 éléments liés par l'intermédiaire d'un atome N, lequel peut contenir un élément de noyau de la série N-R⁵ ou O,
où
R⁵ représente l'hydrogène ou un groupe alkyle en C₁-C₄,
et
R¹⁰ représente l'hydrogène, un groupe alkyle en C₁-C₄, alcoxy en C₁-C₄, cycloalkyle en C₃-C₆, cycloalkyloxy en C₃-C₆ ou -NR¹¹R¹²,
R¹¹ représentant un groupe alkyle en C₁-C₄ ou cycloalkyle en C₃-C₆,
et
R¹² représentant un groupe alkyle en C₁-C₄,
A représente un groupe hétéroaryle à 5 éléments ou un groupe hétérocyclyle à 5 éléments partiellement insaturé,
dans lequel le groupe hétéroaryle et hétérocyclyle sont liés en position 1 ou 2 au noyau phényle et le groupe hétéroaryle et hétérocyclyle présentent eux-mêmes une liaison 1,3 avec le noyau phényle et le groupe carbonylaminométhyle,
et
le groupe hétéroaryle et hétérocyclyle sont substitués avec un substituant R⁶,
où R⁶ est lié sur l'atome voisin de l'atome sur lequel est lié le groupe carbonylaminométhyle et présente une liaison 1,4 par rapport au noyau phényle,
et
où l'atome, sur lequel est lié R⁶, est un atome d'azote ou de carbone,
et
où
R⁶ représente un groupe phényle ou un groupe hétéroaryle à 5 ou 6 éléments,
le groupe phényle et hétéroaryle pouvant être substitués avec de 1 à 3 substituants, les substituants étant choisis indépendamment les uns des autres dans le groupe constitué d'un atome d'halogène, d'un groupe hydroxy, hydroxyméthyle, hydroxyéthyle, amino, aminométhyle, aminoéthyle, alkyle en C₁-C₄, alcoxy en C₁-C₄, alcoxyméthyle en C₁-C₄, alkylamino en C₁-C₄, alkylaminométhyle en C₁-C₄, hydroxycarbonyle, hydroxycarbonylméthyle, aminocarbonyle, aminocarbonylméthyle, alcoxycarbonyle en C₁-C₄, alcoxycarbonylméthyle en C₁-C₄, alkylaminocarbonyle en C₁-C₄, alkylaminocarbonylméthyle en C₁-C₄, aminosulfonyle, alkylaminosulfonyle en C₁-C₄ et alkylsulfonyle en C₁-C₄,
R² représente l'hydrogène, le fluor, le chlore, un groupe cyano, hydroxy, amino, trifluorométhyle, trifluorométhoxy, alkyle en C₁-C₄, alcoxy en C₁-C₄, alcoxyméthyle en C₁-C₄, alkylamino en C₁-C₄, cycloalkyle en C₃-C₆, aminocarbonyle, alcoxycarbonyle en C₁-C₄ ou alkylaminocarbonyle en C₁-C₄,
R³ représente l'hydrogène, le fluor, le chlore, un groupe cyano, hydroxy, amino, trifluorométhyle, trifluorométhoxy, alkyle en C₁-C₄, alcoxy en C₁-C₄, alcoxyméthyle en C₁-C₄, alkylamino en C₁-C₄, cycloalkyle en C₃-C₆, aminocarbonyle, alcoxycarbonyle en C₁-C₄ ou alkylaminocarbonyle en C₁-C₄,
R⁴ représente un groupe de la formule 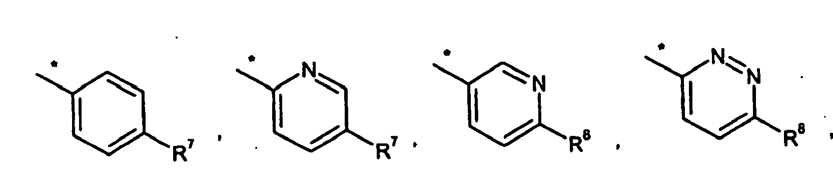 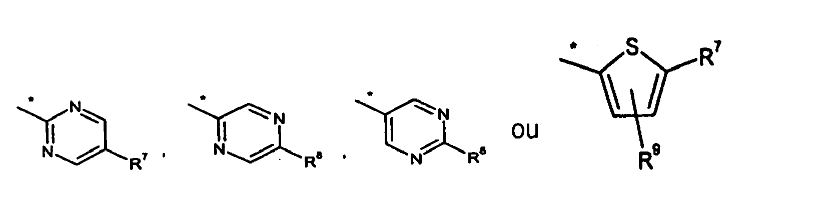 où
* est la position de liaison sur le groupe carbonyle, R⁷ représente l'hydrogène, le fluor, le chlore, un groupe cyano, éthinyle, alkyle en C₁-C₄, alcoxy en C₁-C₄ ou cycloalkyle en C₃-C₆,
R⁸ représente l'hydrogène, un groupe amino, alkyle en C₁-C₄, alkylamino en C₁-C₄ ou cycloalkyle en C₃-C₆,
et
R⁹ représente l'hydrogène, le fluor, le chlore, un groupe amino ou alkyle en C₁-C₄,
où un de ses sels, ses solvates ou des solvates de ses sels.

2. Composé selon la revendication 1, **caractérisé en ce que**
E représente un groupe de la formule 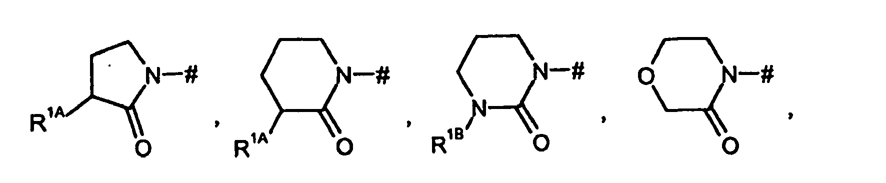 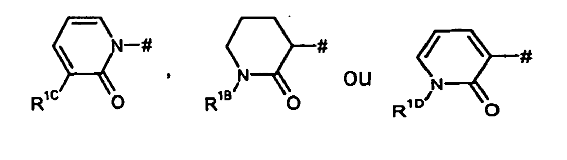 où
# est la position de liaison sur le noyau phényle,
R^{1A} représente l'hydrogène, un groupe hydroxy, hydroxyméthyle, 2-hydroxyéthyle, amino ou méthoxy,
R^{1B} représente l'hydrogène, un groupe hydroxy, amino, méthyle ou éthyle,
dans lequel
le groupe méthyle peut être substitué avec un substituant pyrrolidinin-1-yle, et
le groupe éthyle peut être substitué avec un substituant, le substituant étant choisi dans le groupe constitué d'un groupe hydroxy, amino, diméthylamino et cyclopropylamino,
R^{1C} représente l'hydrogène, un groupe méthyle ou éthyle, dans lequel
le groupe méthyle peut être substitué avec un substituant, le substituant étant choisi dans le groupe constitué d'un groupe hydroxy et pyrrolidin-1-yle,
et
le groupe éthyle peut être substitué avec un substituant, le substituant étant choisi dans le groupe constitué d'un groupe hydroxy, amino et cyclopropylamino,
et
R^{1D} représente l'hydrogène, un groupe méthyle ou éthyle,
dans lequel
le groupe méthyle peut être substitué avec un substituant, le substituant étant choisi dans le groupe constitué du groupe cyclopropylamino et pyrrolidinyl-1-yle,
et
le groupe éthyle peut être substitué avec un substituant, le substituant étant choisi dans le groupe constitué d'un groupe hydroxy, amino et cyclopropylamino,
A représente un groupe hétéroaryle à 5 éléments ou un groupe hétérocyclyle à 5 éléments partiellement insaturé,
dans lequel le groupe hétéroaryle et hétérocyclyle sont liés en position 1 ou 2 au noyau phényle et le groupe hétéroaryle et hétérocyclyle présentent eux-mêmes une liaison 1,3 avec le noyau phényle et le groupe carbonylaminométhyle,
et
le groupe hétéroaryle et hétérocyclyle sont substitués avec un substituant R⁶,
où R⁶ est lié à l'atome voisin de l'atome sur lequel est lié le groupe carbonylaminométhyle et présente une liaison 1,4 au noyau phényle,
et
l'atome, sur lequel est lié R⁶ est un atome d'azote ou de carbone,
et
où
R⁶ représente un groupe phényle ou hétéroaryle à 5 ou 6 éléments,
le groupe phényle et hétéroaryle pouvant être substitués avec de 1 à 3 substituants, les substituants étant choisis indépendamment les uns des autres dans le groupe constitué d'un atome d'halogène, d'un groupe hydroxy, amino, alkyle en C₁-C₄, alcoxy en C₁-C₄, alcoxyméthyle en C₁-C₄, alkylamino en C₁-C₄, alkylaminométhyle en C₁-C₄, hydroxycarbonyle, hydroxycarbonylméthyle, aminocarbonyle, aminocarbonylméthyle, alcoxycarbonyle en C₁-C₄, alcoxycarbonylméthyle en C₁-C₄, alkylaminocarbonyle en C₁-C₄, alkylaminocarbonylméthyle en C₁-C₄, aminosulfonyle, alkylaminosulfonyle en C₁-C₄ et alkylsulfonyle en C₁-C₄,
R² représente l'hydrogène, le fluor, le chlore, un groupe cyano, hydroxy, alkyle en C₁-C₄ ou alcoxy en C₁-C₄,
R³ représente l'hydrogène, le fluor, le chlore, un groupe cyano, hydroxy, alkyle en C₁-C₄, alcoxy en C₁-C₄, alcoxyméthyle en C₁-C₄, cyclopropyle, aminocarbonyle, alcoxycarbonyle en C₁-C₄ ou alkylaminocarbonyle en C₁-C₄,
R⁴ représente un groupe de la formule 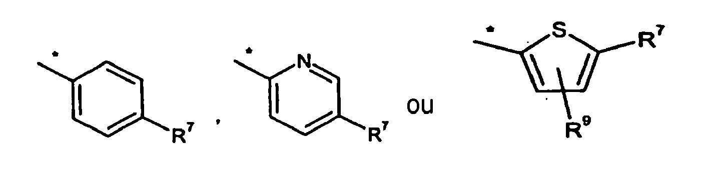 dans lesquelles
* est la position de liaison sur le groupe carbonyle,
R⁷ représente le fluor, le chlore, un groupe éthinyle, méthyle ou méthoxy,
et
R⁹ représente l'hydrogène.

3. Composé selon l'une quelconque des revendications 1 ou 2, **caractérisé en ce que**
E représente un groupe de la formule 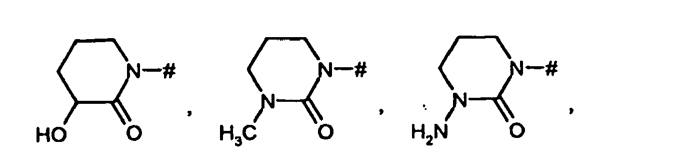 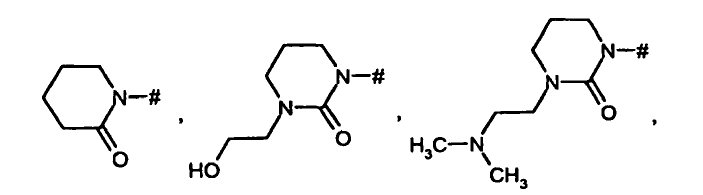 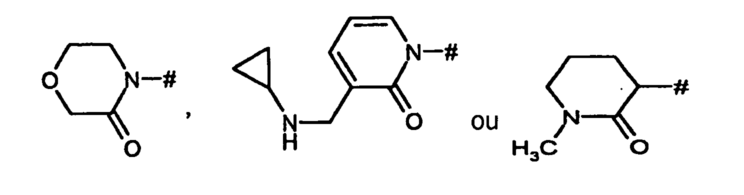 dans lesquelles
# est la position de liaison sur le noyau phényle,
A représente un groupe pyrazolyle, oxadiazolyle ou isoxazolinyle,
dans lequel les groupes pyrazolyle, oxadiazolyle et isoxazolinyle sont liés à la position 1 au noyau phényle et les groupes pyrazolyle, oxadiazolyle et isoxazolinyle présentent eux-mêmes une liaison 1,3 avec le noyau phényle et le groupe carbonylaminométhyle,
et
les groupes pyrazolyle, oxadiazolyle et isoxazolinyle sont substitués avec un substituant R⁶,
où R⁶ est lié à l'atome voisin de l'atome sur lequel est lié le groupe carbonylaminométhyle et présente une liaison 1,4 au noyau phényle
et
l'atome, sur lequel est lié R⁶, est un atome d'azote ou de carbone,
et
où
R⁶ représente un groupe phényle, pyrid-2-yle, pyrid-3-yle, pyrid-4-yle, 1,3-oxazol-2-yle ou pyrimidin-2-yle,
les groupes phényle, pyrid-2-yle, pyrid-3-yle, pyrid-4-yle, 1,3-oxazol-2-yle et pyrimidin-2-yle pouvant être substitués avec de 1 à 3 substituants, les substituants étant choisis indépendamment les uns des autres dans le groupe constitué d'un atome d'halogène, d'un groupe hydroxy, amino, alkyle en C₁-C₄, alcoxy en C₁-C₄, alkylamino en C₁-C₄, hydroxycarbonyle, aminocarbonyle, alcoxycarbonyle en C₁-C₄, alkylaminocarbonyle en C₁-C₄, aminosulfonyle, alkylaminosulfonyle en C₁-C₄ et alkylsulfonyle en C₁-C₄,
R² représente l'hydrogène ou le fluor,
R³ représente l'hydrogène, le fluor, le chlore, un groupe cyano, méthyle, éthyle, n-propyle, iso-propyle, méthoxy, éthoxy, méthoxyméthyle ou cyclopropyle,
R⁴ représente un groupe de la formule 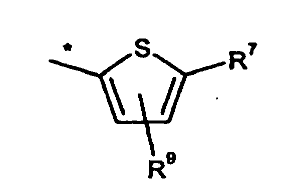 dans laquelle
* est la position de liaison sur le groupe carbonyle,
R⁷ représente le fluor, le chlore ou un groupe méthyle, et
R⁹ représente l'hydrogène.

4. Composé selon l'une quelconque des revendications 1 à 3, **caractérisé en ce que**
E représente un groupe de la formule 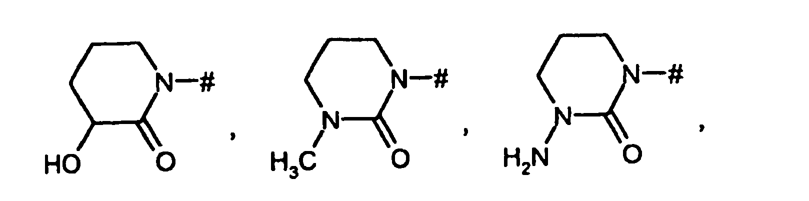 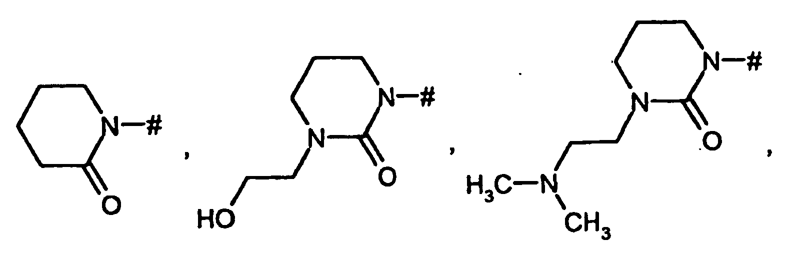 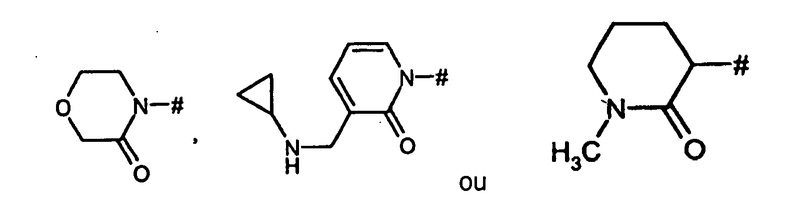 dans lesquelles
# est la position de liaison sur le noyau phényle,
A représente un groupe pyrazolyle, oxadiazolyle ou isoxazolinyle,
dans lequel les groupes pyrazolyle, oxadiazolyle et isoxazolinyle sont liés en position 1 au noyau phényle et les groupes pyrazolyle, oxadiazolyle et isoxazolinyle présentent eux-mêmes une liaison 1,3 avec le noyau phényle et le groupe carbonylaminométhyle,
et
les groupes pyrazolyle, oxadiazolyle et isoxazolinyle sont substitués avec un substituant R⁶,
où R⁶ est lié à l'atome voisin de l'atome sur lequel est lié le groupe carbonylaminométhyle et présente une liaison 1,4 au noyau phényle,
et
l'atome, sur lequel est lié R⁶ est un atome d'azote ou de carbone,
et
où
R⁶ représente un groupe phényle, pyrid-2-yle, pyrid-3-yle ou pyrid-4-yle,
les groupes phényle, pyrid-2-yle, pyrid-3-yle et pyrid-4-yle pouvant être substitués avec un substituant, le substituant étant choisi dans le groupe constitué d'un atome d'halogène, d'un groupe hydroxy, amino, alkyle en C₁-C₄, alcoxy en C₁-C₄, alkylamino en C₁-C₄, hydroxycarbonyle, aminocarbonyle, alcoxycarbonyle en C₁-C₄ et alkylaminocarbonyle en C₁-C₄,
R² représente l'hydrogène ou le fluor,
R³ représente l'hydrogène, le fluor, le chlore, un groupe méthyle ou méthoxy,
R⁴ représente un groupe de la formule 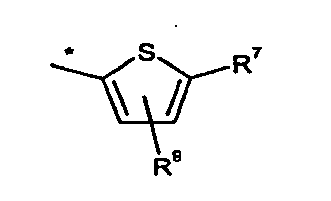 dans laquelle
* est la position de liaison sur le groupe carbonyle,
R⁷ représente le chlore,
et
R⁹ représente l'hydrogène.

5. Procédé pour la préparation d'un composé de la formule (I) ou d'un de ses sels, ses solvates ou des solvates de ses sels selon la revendication 1, **caractérisé en ce que** l'on fait réagir un composé de la formule 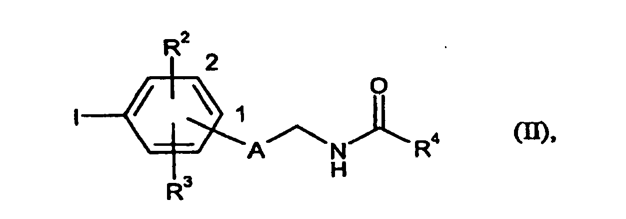 dans laquelle A, R², R³ et R⁴ ont la signification indiquée dans la revendication 1,
avec un composé de la formule
E-H (III),
dans laquelle
E a la signification indiquée dans la revendication 1.

6. Composé selon l'une quelconque des revendications 1 à 4 pour le traitement et/ou la prophylaxie de maladies.

7. Utilisation d'un composé selon l'une quelconque des revendications 1 à 4 pour la préparation d'un médicament destiné au traitement et/ou à la prophylaxie de maladies.

8. Utilisation d'un composé selon l'une quelconque des revendications 1 à 4 pour la préparation d'un médicament destiné au traitement et/ou la prophylaxie de maladies de thrombo-embolies.

9. Médicament contenant un composé selon l'une quelconque des revendications 1 à 4 en combinaison avec un auxiliaire inerte, non toxique, pharmaceutiquement approprié.

10. Médicament contenant un composé selon l'une quelconque des revendications 1 à 4 en combinaison avec un autre principe actif.

11. Médicament selon la revendication 9 ou 10 pour le traitement et/ou la prophylaxie de maladies de thrombo-embolies.
